# EUROPEAN PATENT SPECIFICATION

(11) **EP 3 236 263 B1**
(45) Date of publication and mention of the grant of the patent: **25.12.2019**
(21) Application number: 15847971.7
(22) Date of filing: 01.10.2015
(51) Int. Cl.: G01N 33/68, G01N 33/564

(54) **METHOD FOR DIAGNOSING ARTHROSIS**
VERFAHREN ZUR DIAGNOSE VON ARTHROSE
MÉTHODE DE DIAGNOSTIC DE L'ARTHROSE

(30) Priority: 01.10.2014 ES 201431445
(43) Date of publication of application: 25.10.2017
(73) Proprietor: Servizo Galego De Saúde (Sergas), 15703 Santiago de Compostela (A Coruña) (ES); Universidad de Vigo, 36310 Vigo - Pontevedra (ES); Fundación Profesor Novoa Santos, 15006 A Coruna (ES)
(72) Inventor: BLANCO GARCÍA, Francisco J., E-15703 Santiago de Compostela (A Coruña) (ES); RUIZ ROMERO, Cristina, E-15703 Santiago de Compostela (A Coruña) (ES); FERNÁNDEZ COSTA, Carolina, E-15006 A Coruña (ES); CAPELO MARTÍNEZ, José Luís, E-36310 Vigo (Pontevedra ) (ES); FERNÁNDEZ RIVEROLA, Florentino, E-36310 Vigo (Pontevedra ) (ES); REBOIRO JATO, Miguel, E-36310 Vigo (Pontevedra ) (ES)
(74) Representative: Pons
(86) International application number: PCT/ES2015/070717
(87) International publication number: WO 2016/051006

(56) References cited:
- WO-A1-2011/144934
- WO-A2-2004/082617
- WO-A2-2008/021290
- WO-A2-2010/141469
- TATSUJI NISHIOKA ET AL: "Alpha-1-antitrypsin and complement component C7 are involved in asthma exacerbation", PROTEOMICS - CLINICAL APPLICATIONS, vol. 2, no. 1, 11 January 2008 (2008-01-11), pages 46-54, XP055477310, DE ISSN: 1862-8346, DOI: 10.1002/prca.200780065
- YI-TING CHEN ET AL: "Multiplexed quantification of 63 proteins in human urine by multiple reaction monitoring-based mass spectrometry for discovery of potential bladder cancer biomarkers", JOURNAL OF PROTEOMICS, ELSEVIER, AMSTERDAM, NL, vol. 75, no. 12, 20 December 2011 (2011-12-20), pages 3529-3545, XP028430291, ISSN: 1874-3919, DOI: 10.1016/J.JPROT.2011.12.031 [retrieved on 2012-01-03]
- ANANTH, L. ET AL.: 'Apolipoproteins A-I and B and cholesterol in synovial fluid of patients with rheumatoid arthritis.' METABOLISM CLINICAL AND EXPERIMENTAL. vol. 42, no. 7, July 1993, pages 803 - 806, XP026300552
- DE SENY D. ET AL.: 'Discovery and biochemical characterisation of four novel biomarkers for osteoarthritis.' ANNALS OF THE RHEUMATIC DISEASES. vol. 70, no. 6, June 2011, pages 1144 - 1152, XP002676458
- JMEIAN, Y. ET AL.: 'Micro-high-performance liquid chromatography platform for the depletion of high-abundance proteins and subsequent on-line concentration/capturing of medium- and low-abundance proteins from serum. Application to profiling of protein expression in healthy and osteoarthritis sera by 2-D gel electrophor' ELECTROPHORESIS. vol. 29, no. 13, July 2008, pages 2801 - 2811, XP055424834
- SINZ, A. ET AL.: 'Mass spectrometric proteome analyses of synovial fluids and plasmas from patients suffering from rheumatoid arthritis and comparison to reactive arthritis or osteoarthritis.' ELECTROPHORESIS vol. 23, no. 19, September 2002, pages 3445 - 3456, XP055424841
- QIN, H ET AL.: 'Highly efficient extraction of serum peptides by ordered mesoporous carbon.' ANGEWANDTE CHEMIE vol. 50, no. 51, December 2011, pages 12218 - 12221, XP055424842
- DATABASE UNIPARC DATABASE [Online] 16 September 2011 MEI, X. ET AL.: 'Crystal structure of C-terminal truncated apolipoprotein A-I reveals the assembly of high density lipoprotein (HDL) by dimerizaton.', XP009503002 Retrieved from UNIPARC Database accession no. IPI0002274E97

## Description

The present invention relates to a peptide pattern characteristic of subjects who suffer from arthrosis for use in the diagnosis of said disease. Therefore, the present invention also relates to an *in vitro* diagnostic method and the use of a kit for implementing said diagnostic method. Therefore, the invention belongs to the technical field of disease diagnosis, in particular, the diagnosis of arthrosis.

### STATE OF THE ART

Arthrosis is a rheumatic pathology that damages articular cartilage. Joints are the skeletal components that allow movement and, consequently, functional autonomy, and are formed by the junction of two bones through the articular capsule. In general, there is a fluid called synovial fluid inside them, which is produced by the synovial membrane. The ends of the bones that come together to form the joint are coated with articular cartilage.

When this articular cartilage is injured, it causes pain, rigidity and functional impairment. Normally, arthrosis is located in the cervical and lumbar spine, and in some joints of the shoulder and the fingers, the hip, the knees and the joint of the head of the big toe.

This rheumatic disease is not hereditary, as there is no fixed hereditary pattern as in the case of haemophilia, for example, but it does have a genetic risk component that, together with other factors, may cause it to appear more frequently in subjects with a family history thereof. In Spain, arthrosis affects 10% of the general population, and represents almost one-fourth of all patients who visit rheumatologists.

Currently, the diagnosis of arthrosis is based on the evaluation of the symptoms and the physical examination of the patients by the physicians. They assess the patients' symptoms, where are they located, what type of pain they have, under which circumstances it improves (e.g., with rest) or gets worse (when going upstairs or downstairs, when opening or closing water taps, etc.), etc. They also ask about other diseases that the patients may have, what treatments are they receiving, and whether or not they or any relatives suffer, or have suffered, from any type of rheumatic disease, trauma or prior articular cartilage injury.

X-rays make it possible to confirm the diagnosis of arthrosis, since they show the radiological changes in the joints typical of arthrotic processes. Radiological studies may be used to determine the severity of arthrosis in a more precise manner.

Blood tests have no utility for diagnosing arthrosis. All the results determined are always normal, including the so-called "rheumatic evidence", i.e. they do not help to conclude that said subjects suffer from the disease. The only advantage of performing said tests is to confirm the diagnosis of arthrosis when they are normal, since this makes it possible to discard other rheumatic diseases that do produce some alterations in the laboratory tests. For example, in arthritis, the erythrocyte sedimentation rate, the rheumatoid factor and other rheumatic evidences are altered; in gout, uric acid levels are high, etc. Thus far, no diagnostic markers have been identified in the patients' serum or synovial fluid that allows the diagnosis or follow-up of arthrosis.

In this sense, WO2010141469A2 relates to a method for characterizing the risk a subject will develop an autoimmune and/or alloimmune disease, preferably an idiopathic pneumonia (IPS) following tissue transplant, and to predict the response of the patient to an anti-TNF treatment, by the use of expression levels of markers including apolipoprotein A-IV, Zn-alpha-2-glycoprotein, apolipoprotein A-1 and alpha-2-antiplasmin.

Moreover, WO2008021290A2 relates to organ-specific proteins and polynucleotides that encode them, together the diagnostic and prognostic panels, sets and individual agents comprising reagents or probes to detect the mentioned organ-specific proteins or polynucleotides and methods of identifying and using organ-specific proteins. Particularly, this document discloses panels of proteins that have been identified as related with specific organs, and moreover, also relate these organs with specific organ-related diseases, such as bladder-related proteins could be putatively related with bladder cancer.

In addition, WO2011144934A1 discloses the use of a specific set of biomarkers, such as, complement factor H, ApoAI, A2AP, ApoA4, ZA2Gs, etc., for the diagnosis, therapeutic monitoring or predisposition to schizophrenia and other psychotic disorders.

Furthermore, Nishioka T. *et al.* (Nishioka t. et al. Proteomics Clin Appl. 2008 Jan;2(1):46-54) refers to a proteomic analysis for the identification of proteins biomarkers (APOA1, APOA4, ZA2G and A2AP) involved in asthma exacerbation.

Additionally, Chen YT. *et al.* (Chen YT. et al. J Proteomics. 2012 Jun 27;75(12):3529-45) relate to the identification of 63 proteins such as, APOA1, APOA4, ZA2G and A2AP, among other, in an isolated urine sample from a subject which can be useful as diagnostic biomarkers for bladder cancer.

These documents relate to the use of the expression levels of biomarkers in the diagnosis of different diseases, but none of them refers to the diagnosis of arthrosis.

However, significant progress is being made in the study of the so-called biological markers of arthrosis. These are products released from the articular cartilage into the synovial fluid, the urine or the blood during the synthesis and destruction of the cartilage. Some of them are derivatives of keratan sulfate, chondroitin sulfate, hyaluronic acid or the cartilage oligomeric matrix protein (COMP). Increased knowledge about the biochemical markers of arthrosis would allow for a better diagnosis and earlier intervention in the treatment of the disease, in addition to facilitating the development of drugs, initiating improved therapies, allowing for better medicament options, offering safe dosage options and, finally, reducing health care costs. Consequently, there is a need in the state of the art to identify markers of arthrosis that may allow for an early diagnosis of the disease in a simple, reliable, safe manner.

### DESCRIPTION OF THE INVENTION

The authors of the present invention have discovered that subjects who suffer from arthrosis present a peptide pattern that is not present in healthy subjects, or in subjects who suffer from another type of rheumatic disease, such as psoriatic arthritis (PsA) and rheumatoid arthritis (RA), which makes said peptide pattern a useful tool for the specific diagnosis of arthrosis. In order to obtain the aforementioned pattern, the authors started with serum samples from patients diagnosed with arthrosis and performed a chemical depletion with dithiothreitol (DTT) and acetonitrile (ACN), in order to subsequently perform a tryptic digestion accelerated by means of ultrasound. Thereafter, the resulting peptides were retained in a commercial C18 tip and eluted with different amounts of ACN. Finally, each eluted fraction was analysed in quintuplicate by means of MALDI-TOF/TOF mass spectrometry (Example 1), to obtain the masses of the peptides characteristic of the peptide pattern present in subjects who suffer from arthrosis. The subsequent validation of the peptide pattern obtained (Example 2) confirmed the utility of this pattern for the diagnosis of arthrosis.

The different inventive aspects developed by the inventors are described below.

In a first aspect the present invention relates to the simultaneous *in vitro* use of the expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, as a marker for the diagnosis of arthrosis in a biological sample isolated from a subject.

In a preferred embodiment, the simultaneous *in vitro* use further comprising the expression level and/or quantity of at least one of the peptides selected from the list consisting of: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof.

In another preferred embodiment, the simultaneous *in vitro* use it is characterized in that the isolated biological sample is a biological fluid, preferably selected from the group consisting of blood, serum, plasma, synovial fluid and urine.

In another aspect, the present invention relates to a method *in vitro* for the diagnosis of arthrosis in an isolated sample of a subject, which comprises:
(a) simultaneously detecting, in the isolated biological sample of the subject, the expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, and
(b) comparing the expression level and/or quantity obtained in step (a) with a reference quantity from an isolated biological sample obtained from a healthy control subject,
wherein a significantly higher expression level and/or quantity of the peptides in the isolated biological sample from the subject of step (a) with respect to the expression level and/or quantity of the peptides in the isolated biological sample from the healthy control subject, is indicative of arthrosis.

In a preferred embodiment, the method *in vitro* of the invention, further comprising determining, in step (a), the expression level and/or quantity of at least one of the peptides selected from the list consisting of: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, or combinations thereof, wherein a significantly higher expression level and/or quantity of at least one of the peptides selected from the list consisting of: SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77, SEQ ID NO: 78, or any combinations thereof, and/or a significantly lower expression level and/or quantity of at least one of the peptides selected from the list consisting of: SEQ ID NO: 12, SEQ ID NO: 34, SEQ ID NO: 50, SEQ ID NO: 60, SEQ ID NO: 62, SEQ ID NO: 64, SEQ ID NO: 74, or any combinations thereof, in the isolated biological sample from the subject of step (a) with respect to the expression level and/or quantity of the peptides in the isolated biological sample from the healthy control subject, is indicative of arthrosis.

In another preferred embodiment, the method *in vitro* of the invention it is characterised in that the isolated biological sample is a biological fluid, preferably selected from the group consisting of blood, serum, plasma, synovial fluid and urine.

In another preferred embodiment of the method *in vitro* of the invention, it is characterized in that the detection of the expression levels and/or quantity of the peptides is performed by means of mass spectrometry, ELISA, array, microarray, flow cytometry, mass spectrometry, gas chromatography-mass spectrometry or liquid chromatography-mass spectrometry.

In another aspect, the present invention also relates to the use of a kit for the *in vitro* diagnosis of arthrosis in an isolated biological sample from a subject, wherein the kit comprises specific antibodies against the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, or specific oligonucleotides capable of hybridising to the nucleotide sequence that encodes the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

In a preferred embodiment, the use of the kit of the invention further comprises at least one specific antibody against at least one of the peptides with the sequences SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof, or at least one specific oligonucleotide capable of hybridising to the nucleotide sequence that encodes at least one of the peptides with the sequences SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof.

In another preferred embodiment, the use of the kit of the invention it is characterized in that the isolated biological sample is a biological fluid, preferably selected from the group consisting of blood, serum, plasma, synovial fluid and urine.

### Summary of other aspects of the disclosure.

The authors of the present description have discovered that patients with arthrosis present a characteristic peptide pattern that is not present neither in subjects with rheumatic diseases other than arthrosis (such as psoriatic arthritis or rheumatoid arthritis), nor in healthy subjects.

Therefore, one aspect of the disclosure relates to a peptide pattern, hereinafter peptide pattern of the disclosure, that comprises the following peptides: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

In the present disclosure, "peptide pattern" is understood to mean the set or group of peptides present in a biological sample from a subject that is characteristic of his/her physiological state. Within the context of the present disclosure, the peptide pattern described is characteristic of subjects who suffer from arthrosis.

In the present disclosure, "peptide" is understood to mean the molecule formed by the binding of several amino acids through peptide bonds which have, approximately, between 2 and 100 amino acids. As a guideline, if the peptide comprises between approximately 2 and 15 amino acids, it is called oligopeptide, and, if it comprises between approximately 16 and 100 amino acids, it is called polypeptide.

As shown in the examples included in the present description, the peptide pattern of the disclosure was obtained by means of MALDI-TOF/TOF mass spectrometry, reason, in addition to being characterized by their specific amino acid sequence, the peptides of the disclosure are also characterized by their mass/charge value and their molecular weight [see **Table 1**], which are standard parameters in this type of technique.

**Table 1. Mass/charge ratio values and molecular weights obtained by means of mass spectrometry for each peptide comprised within the peptide pattern of the disclosure. Table 1 also shows the peptide sequence of the peptides that comprise the pattern of the disclosure.**

| | **Mass/charge (m/z)** | **Molecular weight (Da)** | **SEQ ID NO:** | **Peptide sequence** |
|---|---|---|---|---|
| Peptide 01 | 1392.7526 | 1391.7526 | 1 | QLTPYAQRMER |
| Peptide 02 | 1532.8060 | 1531.806 | 2 | QKWEAEPVYVQR |
| Peptide 03 | 1758.9790 | 1757.979 | 3 | ELQQRLEPYADQLR |
| Peptide 04 | 1910.0176 | 1909.0176 | 4 | LHELQEKLSPLGEEMR |
| Peptide 05 | 2165.1272 | 2164.1272 | 5 | QKLHELQEKLSPLGEEMR |
| Peptide 06 | 2180.1445 | 2179.1445 | 6 | LHELQEKLSPLGEEMRDR |
| Peptide 07 | 2280.1501 | 2279.1501 | 7 | DSFHLDEQFTVPVEMMQAR |
| Peptide 08 | 2291.2260 | 2290.226 | 8 | ARAHVDALRTHLAPYSDELR |
| Peptide 09 | 2407.2880 | 2406.288 | 9 | LHELQEKLSPLGEEMRDRAR |
| Peptide 10 | 2436.2922 | 2435.2922 | 10 | QKLHELQEKLSPLGEEMRDR |
| Peptide 11 | 2663.4314 | 2662.4314 | 11 | QKLHELQEKLSPLGEEMRDRAR |

In a particular aspect, the peptide pattern of the disclosure further comprises at least one peptide selected from the group consisting of the peptides shown in **Table 2,** which are characterized by both their specific amino acid sequence, and their mass/charge value and molecular weight.

**Table 2. Sequences, mass/charge values and molecular weights obtained by means of mass spectrometry for each additional peptide that may be used in combination with the peptide pattern of the disclosure.**

| | **Mass/charge (m/z)** | **Molecular weight (Da)** | **SEQ ID NO:** | **Peptide sequence** |
|---|---|---|---|---|
| Peptide 12 | 871.502 | 870.502 | 12 | VLNQELR |
| Peptide 13 | 896.490 | 895.490 | 13 | YLTWASR |
| Peptide 14 | 961.627 | 960.627 | 14 | GVTFLLRR |
| Peptide 15 | 980.529 | 979.529 | 15 | VGYVSGWGR |
| Peptide 16 | 1019.604 | 1018.604 | 16 | AFKAWAVAR |
| Peptide 17 | 1108.588 | 1107.588 | 17 | LQTDLSEFR |
| Peptide 18 | 1117.669 | 1116.669 | 18 | GFSPKDVLVR |
| Peptide 19 | 1148.634 | 1147.634 | 19 | DAVSAAFKGLK |
| Peptide 20 | 1213.667 | 1212.667 | 20 | WLQGSQELPR |
| Peptide 21 | 1237.674 | 1236.674 | 21 | LETPDFQLFK |
| Peptide 22 | 1247.652 | 1246.652 | 22 | LGMFNIQHCK |
| Peptide 23 | 1249.655 | 1248.655 | 23 | LICQATGFSPR |
| Peptide 24 | 1255.716 | 1254.716 | 24 | LRCLAPLEGAR |
| Peptide 25 | 1263.683 | 1262.683 | 25 | LGMFNIQHCK |
| Peptide 26 | 1299.609 | 1298.609 | 26 | WQEEMELYR |
| Peptide 27 | 1380.780 | 1379.780 | 27 | VQPYLDDFQKK |
| Peptide 28 | 1403.749 | 1402.749 | 28 | RLGMFNIQHCK |
| Peptide 29 | 1449.800 | 1448.800 | 29 | VVAGVANALAHKYH |
| Peptide 30 | 1465.689 | 1464.689 | 30 | DSGEGDFLAEGGGVR |
| Peptide 31 | 1467.852 | 1466.852 | 31 | VEPLRAELQEGAR |
| Peptide 32 | 1470.813 | 1469.813 | 32 | WLQGSQELPREK |
| Peptide 33 | 1502.810 | 1501.810 | 33 | VCPFAGILENGAVR |
| Peptide 34 | 1518.710 | 1517.710 | 34 | ADSGEGDFLAEGGGVR |
| Peptide 35 | 1519.750 | 1518.750 | 35 | ALYLQYTDETFR |
| Peptide 36 | 1568.914 | 1567.914 | 36 | LAARLEALKENGGAR |
| Peptide 37 | 1569.907 | 1568.907 | 37 | LAARLEALKENGGAR |
| Peptide 38 | 1585.846 | 1584.846 | 38 | THLAPYSDELRQR |
| Peptide 39 | 1612.849 | 1611.849 | 39 | LLDNWDSVTSTFSK |
| Peptide 40 | 1641.892 | 1640.892 | 40 | ITPNLAEFAFSLYR |
| Peptide 41 | 1641.892 | 1640.892 | 41 | AGDFLEANYMNLQR |
| Peptide 42 | 1669.862 | 1668.862 | 42 | VLGAFSDGLAHLDNLK |
| Peptide 43 | 1706.931 | 1705.931 | 43 | QKVEPLRAELQEGAR |
| Peptide 44 | 1707.875 | 1706.875 | 44 | YVMLPVADQDQCIR |
| Peptide 45 | 1736.970 | 1735.970 | 45 | LSQRFPKAEFAEVSK |
| Peptide 46 | 1743.882 | 1742.882 | 46 | YAASSYLSLTPEQWK |
| Peptide 47 | 1745.901 | 1744.901 | 47 | YVGGQEHFAHLLILR |
| Peptide 48 | 1771.885 | 1770.885 | 48 | SLAELGGHLDQQVEEF |
| Peptide 49 | 1786.909 | 1785.909 | 49 | GLPGPPDVPDHAAYHPF |
| Peptide 50 | 1810.992 | 1809.992 | 50 | SYFEKSKEQLTPLIK |
| Peptide 51 | 1815.922 | 1814.922 | 51 | DSGRDYVSQFEGSALGK |
| Peptide 52 | 1825.005 | 1824.005 | 52 | ILGGHLDAKGSFPWQAK |
| Peptide 53 | 1834.946 | 1833.946 | 53 | VMPICLPSKDYAEVGR |
| Peptide 54 | 1864.027 | 1863.027 | 54 | EVQLVESGGGLVQPGGSLR |
| Peptide 55 | 1875.997 | 1874.997 | 55 | VYACEVTHQGLSSPVTK |
| Peptide 56 | 1876.017 | 1875.017 | 56 | VTLTCVAPLSGVDFQLR |
| Peptide 57 | 1884.000 | 1883.000 | 57 | EIVLTQSPGTLSLSPGER |
| Peptide 58 | 1899.060 | 1898.060 | 58 | FNKPFVFLMIEQNTK |
| Peptide 59 | 1906.873 | 1905.873 | 59 | SEAEDASLLSFMQGYMK |
| Peptide 60 | 1922.877 | 1921.877 | 60 | SEAEDASLLSFMQGYMK |
| Peptide 61 | 1932.038 | 1931.038 | 61 | SLHTLFGDKLCTVATLR |
| Peptide 62 | 2012.011 | 2011.011 | 62 | TFGSGEADCGLRPLFEKK |
| Peptide 63 | 2020.138 | 2019.138 | 63 | VLDAVRGSPAINVAVHVFR |
| Peptide 64 | 2153.112 | 2152.112 | 64 | SLEDKTERELLESYIDGR |
| Peptide 65 | 2186.092 | 2185.092 | 65 | LYHSEAFTVNFGDTEEAKK |
| Peptide 66 | 2190.132 | 2189.132 | 66 | CEGPIPDVTFELLREGETK |
| Peptide 67 | 2296.219 | 2295.219 | 67 | TPGAAANLELIFVGPQHAGNYR |
| Peptide 68 | 2315.195 | 2314.195 | 68 | QSNNKYAASSYLSLTPEQWK |
| Peptide 69 | 2344.169 | 2343.169 | 69 | SEAEDASLLSFMQGYMKHATK |
| Peptide 70 | 2348.245 | 2347.245 | 70 | AHVDALRTHLAPYSDELRQR |
| Peptide 71 | 2394.298 | 2393.298 | 71 | LTPYADEFKVKIDQTVEELR |
| Peptide 72 | 2409.281 | 2408.281 | 72 | |
| Peptide 73 | 2565.322 | 2564.322 | 73 | |
| Peptide 74 | 2602.358 | 2601.358 | 74 | |
| Peptide 75 | 2652.448 | 2651.448 | 75 | |
| Peptide 76 | 2670.387 | 2669.387 | 76 | DEPPQSPWDRVKDLATVYVDVLK |
| Peptide 77 | 2695.362 | 2694.362 | 77 | QSNNKYAASSYLSLTPEQWKSHR |
| Peptide 78 | 2790.425 | 2789.425 | 78 | YRSWVPHTFESELSDPVELLVAES |
| Peptide 79 | 2940.755 | 2939.755 | 79 | |

In another, more particular aspect, the peptide pattern of the disclosure further comprises at least one peptide selected from the group consisting of the peptides shown in **Table 3**, which are characterized by both their specific amino acid sequence, and their mass/charge value and molecular weight. The peptides shown in Table 3 may be used in combination with the peptide pattern of the disclosure in order to differentiate between arthrotic subjects and normal subjects. In another, more preferred aspect, arthrotic subjects show an increased presence of the peptides shown in Table 3 as compared to the healthy control subjects.

**Table 3. Sequences, mass/charge values and molecular weights obtained by means of mass spectrometry for each additional peptide that may be used in combination with the peptide pattern of the disclosure in order to differentiate between arthrotic subjects and normal subjects.**

| | **Mass/charge (m/z)** | **Molecular weight (Da)** | **SEQ ID NO:** | **Peptide sequence** |
|---|---|---|---|---|
| Peptide 13 | 896.490 | 895.490 | 13 | YLTWASR |
| Peptide 14 | 961.627 | 960.627 | 14 | GVTFLLRR |
| Peptide 15 | 980.529 | 979.529 | 15 | VGYVSGWGR |
| Peptide 16 | 1019.604 | 1018.604 | 16 | AFKAWAVAR |
| Peptide 17 | 1108.588 | 1107.588 | 17 | LQTDLSEFR |
| Peptide 18 | 1117.669 | 1116.669 | 18 | GFSPKDVLVR |
| Peptide 19 | 1148.634 | 1147.634 | 19 | DAVSAAFKGLK |
| Peptide 20 | 1213.667 | 1212.667 | 20 | WLQGSQELPR |
| Peptide 21 | 1237.674 | 1236.674 | 21 | LETPDFQLFK |
| Peptide 24 | 1255.716 | 1254.716 | 24 | LRCLAPLEGAR |
| Peptide 28 | 1403.749 | 1402.749 | 28 | RLGMFNIQHCK |
| Peptide 32 | 1470.813 | 1469.813 | 32 | WLQGSQELPREK |
| Peptide 33 | 1502.810 | 1501.810 | 33 | VCPFAGILENGAVR |
| Peptide 36 | 1568.914 | 1567.914 | 36 | LAARLEALKENGGAR |
| Peptide 38 | 1585.846 | 1584.846 | 38 | THLAPYSDELRQR |
| Peptide 40 | 1641.892 | 1640.892 | 40 | ITPNLAEFAFSLYR |
| Peptide 41 | 1641.892 | 1640.892 | 41 | AGDFLEANYMNLQR |
| Peptide 42 | 1669.862 | 1668.862 | 42 | VLGAFSDGLAHLDNLK |
| Peptide 43 | 1706.931 | 1705.931 | 43 | QKVEPLRAELQEGAR |
| Peptide 51 | 1815.922 | 1814.922 | 51 | DSGRDYVSQFEGSALGK |
| Peptide 52 | 1825.005 | 1824.005 | 52 | ILGGHLDAKGSFPWQAK |
| Peptide 53 | 1834.946 | 1833.946 | 53 | VMPICLPSKDYAEVGR |
| Peptide 54 | 1864.027 | 1863.027 | 54 | EVQLVESGGGLVQPGGSLR |
| Peptide 55 | 1875.997 | 1874.997 | 55 | VYACEVTHQGLSSPVTK |
| Peptide 56 | 1876.017 | 1875.017 | 56 | VTLTCVAPLSGVDFQLR |
| Peptide 57 | 1884.000 | 1883.000 | 57 | EIVLTQSPGTLSLSPGER |
| Peptide 65 | 2186.092 | 2185.092 | 65 | LYHSEAFTVNFGDTEEAKK |
| Peptide 67 | 2296.219 | 2295.219 | 67 | TPGAAANLELIFVGPQHAGNYR |
| Peptide 68 | 2315.195 | 2314.195 | 68 | QSNNKYAASSYLSLTPEQWK |
| Peptide 70 | 2348.245 | 2347.245 | 70 | AHVDALRTHLAPYSDELRQR |
| Peptide 75 | 2652.448 | 2651.448 | 75 | |
| Peptide 76 | 2670.387 | 2669.387 | 76 | DEPPQSPWDRVKDLATVYVDVLK |
| Peptide 77 | 2695.362 | 2694.362 | 77 | QSNNKYAASSYLSLTPEQWKSHR |
| Peptide 78 | 2790.425 | 2789.425 | 78 | YRSWVPHTFESELSDPVELLVAES |

In another, more preferred aspect, the peptide pattern of the disclosure further comprises at least one peptide selected from the group consisting of the peptides shown in **Table 4**, which are characterized by both their specific amino acid sequence, and their mass/charge value and molecular weight. The peptides shown in Table 4 may be used in combination with the peptide pattern of the disclosure in order to differentiate between arthrotic subjects and normal subjects. In another preferred aspect, arthrotic subjects show a decreased presence of the peptides shown in Table 4 as compared to the healthy control subjects.

**Table 4. Sequences and mass/charge values and molecular weights obtained by means of mass spectrometry for each additional peptide that may be used in combination with the peptide pattern of the disclosure in order to differentiate between arthrotic subjects and normal subjects.**

| | **Mass/charge (m/z)** | **Molecular weight (Da)** | **SEQ ID NO:** | **Peptide sequence** |
|---|---|---|---|---|
| Peptide 12 | 871.502 | 870.502 | 12 | VLNQELR |
| Peptide 34 | 1518.71 | 1517.71 | 34 | ADSGEGDFLAEGGGVR |
| Peptide 50 | 1810.992 | 1809.992 | 50 | SYFEKSKEQLTPLIK |
| Peptide 60 | 1922.877 | 1921.877 | 60 | SEAEDASLLSFMQGYMK |
| Peptide 62 | 2012.011 | 2011.011 | 62 | TFGSGEADCGLRPLFEKK |
| Peptide 64 | 2153.112 | 2152.112 | 64 | SLEDKTERELLESYIDGR |
| Peptide 74 | 2602.358 | 2601.358 | 74 | |

In another particular aspect, the peptide pattern of the disclosure further comprises at least one peptide selected from the group consisting of the peptides shown in **Table 5,** which are characterized by both their specific amino acid sequence, and their mass/charge value and molecular weight. The peptides shown in Table 5 may be used in combination with the peptide pattern of the disclosure in order to differentiate between arthrotic subjects and subjects suffering from rheumatoid arthritis.

**Table 5. Sequences, mass/charge values and molecular weights obtained by means of mass spectrometry for each additional peptide that may be used in combination with the peptide pattern of the disclosure in order to differentiate between arthrotic subjects and subjects suffering from rheumatoid arthritis.**

| | **Mass/charge (m/z)** | **Molecular weight (Da)** | **SEQ ID NO:** | **Peptide sequence** |
|---|---|---|---|---|
| Peptide 22 | 1247.652 | 1246.652 | 22 | LGMFNIQHCK |
| Peptide 23 | 1249.655 | 1248.655 | 23 | LICQATGFSPR |
| Peptide 26 | 1299.609 | 1298.609 | 26 | WQEEMELYR |
| Peptide 30 | 1465.689 | 1464.689 | 30 | DSGEGDFLAEGGGVR |
| Peptide 31 | 1467.852 | 1466.852 | 31 | VEPLRAELQEGAR |
| Peptide 35 | 1519.75 | 1518.75 | 35 | ALYLQYTDETFR |
| Peptide 39 | 1612.849 | 1611.849 | 39 | LLDNWDSVTSTFSK |
| Peptide 45 | 1736.97 | 1735.97 | 45 | LSQRFPKAEFAEVSK |
| Peptide 46 | 1743.882 | 1742.882 | 46 | YAASSYLSLTPEQWK |
| Peptide 47 | 1745.901 | 1744.901 | 47 | YVGGQEHFAHLLILR |
| Peptide 48 | 1771.885 | 1770.885 | 48 | SLAELGGHLDQQVEEF |
| Peptide 58 | 1899.060 | 1898.060 | 58 | FNKPFVFLMIEQNTK |
| Peptide 59 | 1906.873 | 1905.873 | 59 | SEAEDASLLSFMQGYMK |
| Peptide 61 | 1932.038 | 1931.038 | 61 | SLHTLFGDKLCTVATLR |
| Peptide 63 | 2020.138 | 2019.138 | 63 | VLDAVRGSPAINVAVHVFR |
| Peptide 66 | 2190.132 | 2189.132 | 66 | CEGPIPDVTFELLREGETK |
| Peptide 69 | 2344.169 | 2343.169 | 69 | SEAEDASLLSFMQGYMKHATK |
| Peptide 71 | 2394.298 | 2393.298 | 71 | LTPYADEFKVKIDQTVEELR |
| Peptide 72 | 2409.281 | 2408.281 | 72 | |
| Peptide 73 | 2565.322 | 2564.322 | 73 | |

In another particular aspect, the peptide pattern of the disclosure further comprises at least one peptide selected from the group consisting of the peptides shown in **Table 6,** which are characterized by both their specific amino acid sequence, and their mass/charge value and molecular weight. The peptides shown in Table 6 may be used in combination with the peptide pattern of the disclosure in order to differentiate between arthrotic subjects and subjects suffering from psoriatic arthritis.

**Table 6. Sequences, mass/charge values and molecular weights obtained by means of mass spectrometry for each additional peptide that may be used in combination with the peptide pattern of the disclosure in order to differentiate between arthrotic subjects and subjects suffering from psoriatic arthritis.**

| | **Mass/charge (m/z)** | **Molecular weight (Da)** | **SEQ ID NO:** | **Peptide sequence** |
|---|---|---|---|---|
| Peptide 22 | 1247.652 | 1246.652 | 22 | LGMFNIQHCK |
| Peptide 25 | 1263.683 | 1262.683 | 25 | LGMFNIQHCK |
| Peptide 26 | 1299.609 | 1298.609 | 26 | WQEEMELYR |
| Peptide 27 | 1380.78 | 1379.78 | 27 | VQPYLDDFQKK |
| Peptide 29 | 1449.8 | 1448.8 | 29 | VVAGVANALAHKYH |
| Peptide 30 | 1465.689 | 1464.689 | 30 | DSGEGDFLAEGGGVR |
| Peptide 31 | 1467.852 | 1466.852 | 31 | VEPLRAELQEGAR |
| Peptide 37 | 1569.907 | 1568.907 | 37 | LAARLEALKENGGAR |
| Peptide 39 | 1612.849 | 1611.849 | 39 | LLDNWDSVTSTFSK |
| Peptide 46 | 1743.882 | 1742.882 | 46 | YAASSYLSLTPEQWK |
| Peptide 47 | 1745.901 | 1744.901 | 47 | YVGGQEHFAHLLILR |
| Peptide 48 | 1771.885 | 1770.885 | 48 | SLAELGGHLDQQVEEF |
| Peptide 49 | 1786.909 | 1785.909 | 49 | GLPGPPDVPDHAAYHPF |
| Peptide 58 | 1899.060 | 1898.060 | 58 | FNKPFVFLMIEQNTK |
| Peptide 59 | 1906.873 | 1905.873 | 59 | SEAEDASLLSFMQGYMK |
| Peptide 66 | 2190.132 | 2189.132 | 66 | CEGPIPDVTFELLREGETK |
| Peptide 69 | 2344.169 | 2343.169 | 69 | SEAEDASLLSFMQGYMKHATK |
| Peptide 79 | 2940.755 | 2939.755 | 79 | |

The methods and techniques used to obtain peptide patterns from a sample are widely known in the state of the art, and will be explained in detail in the description of the diagnostic method of the disclosure.

Another aspect of the disclosure relates to the use of the peptide pattern of the disclosure as a diagnostic marker for arthrosis in a subject.

Thus, another object of the present disclosure relates to the simultaneous *in vitro* use of the expression level of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, as a marker designed to obtain useful data for the diagnosis and classification of rheumatic diseases in an subject.

In a preferred aspect the *in vitro* use of the peptide pattern of the disclosure is characterized in that it further comprises determining the expression level of at least one of the peptides selected from the list consisting of: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof.

In another preferred aspect, the *in vitro* use of the peptide pattern of the disclosure is characterized in that the rheumatic diseases are selected from any of the following: arthrosis, rheumatoid arthritis and psoriatic arthritis.

Another aspect of the present disclosure relates to a method for obtaining useful data, hereinafter called first method of the disclosure, for the diagnosis and classification of rheumatic diseases, which comprises:
(a) obtaining an isolated biological sample from a subject,
(b) simultaneously detecting, in the isolated biological sample of (a), the expression level of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

In a preferred aspect, the first method of the disclosure further comprises determining, in step (b), the expression level and/or the quantity of at least one of the peptides selected from the list consisting of: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof.

In another preferred aspect, the first method of the disclosure is characterized in that the rheumatic diseases are selected from any of the following: arthrosis, rheumatoid arthritis and psoriatic arthritis.

Another aspect of the present disclosure relates to a diagnostic and classification method for arthrotic diseases, hereinafter called second method of the disclosure, which comprises steps (a) and (b) according to the first method of the disclosure, and further comprises:
(c) comparing the quantities obtained in step (b) for the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77 and SEQ ID NO: 78, with a reference quantity from a sample obtained from a healthy control subject, and
(d) assigning the subject of step (a) to the group of subject suffering from arthrosis when they present:
   (i) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, detected in (b), or
   (ii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and SEQ ID NO: 11, and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, detected in (b); or
   (iii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77, and SEQ ID NO: 78, and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, detected in (b),
as compared to the expression level and/or the quantity of the same peptides detected in a reference sample obtained from a healthy control subject.

Another object of the present disclosure relates to a diagnostic and classification method for arthrotic diseases, hereinafter third method of the disclosure, which comprises steps (a) and (b) according to the first method of the disclosure and further comprises:
(c) comparing the quantities obtained in step (b) for the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 34, SEQ ID NO: 50, SEQ ID NO: 60, SEQ ID NO: 62, SEQ ID NO: 64 and SEQ ID NO: 74, SEQ ID NO: 22, SEQ ID NO: 23, SEQ ID NO: 26, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 61, SEQ ID NO: 63, SEQ ID NO: 66, SEQ ID NO: 71, SEQ ID NO: 72 and SEQ ID NO: 73, SEQ ID NO: 30, SEQ ID NO: 59 and SEQ ID NO: 69, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, with a reference quantity from a sample obtained from a subject suffering from arthrosis, and
(d) assigning the subject of step (a) to the group of subjects suffering from rheumatoid arthritis when they present:
   (i) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6 SEQ ID NO: 7, SEQ ID NO: 8, SEQ ID NO: 9, SEQ ID NO: 10, SEQ ID NO: 11, SEQ ID NO: 22, SEQ ID NO: 23, SEQ ID NO: 26, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 61, SEQ ID NO: 63, SEQ ID NO: 66, SEQ ID NO: 71, SEQ ID NO: 72 and SEQ ID NO: 73, detected in (b); or
   (ii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and a significantly lower expression level and/or quantity of the peptides with the sequences SEQ ID NO: 30, SEQ ID NO: 59 and SEQ ID NO: 69, detected in (b); or
   (iii) a significantly higher expression level and/or quantity of the peptides of step (i), and the peptides with the sequences SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, detected in (b); or
   (iv) a significantly higher expression level and/or quantity of the peptides of step (i), and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, detected in (b); or
   (v) a significantly higher expression level and/or quantity of the peptides of step (iii), and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, detected in (b); or
   (vi) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, and a significantly lower expression level and/or quantity of the peptides with the sequences SEQ ID NO: 30, SEQ ID NO: 59 and SEQ ID NO: 69, detected in (b); or
   (vii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and SEQ ID NO: 11, and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, SEQ ID NO: 30, SEQ ID NO: 59 and SEQ ID NO: 69, detected in (b); or
   (viii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64, SEQ ID NO: 74, SEQ ID NO: 30, SEQ ID NO: 59 and SEQ ID NO: 69,
as compared to the expression level and/or the quantity of the same peptides detected in a reference sample obtained from a subject suffering from arthrosis.

Another aspect of the present disclosure relates to a diagnostic and classification method for arthrotic diseases, hereinafter called fourth method of the disclosure, which comprises steps (a) and (b) according to the first method of the disclosure, and further comprises:
(c) comparing the quantities obtained in step (b) for the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 22, SEQ ID NO: 26, SEQ ID NO: 27, SEQ ID NO: 31, SEQ ID NO: 37, SEQ ID NO: 39, SEQ ID NO: 46, SEQ ID NO: 47, SEQ ID NO: 48, SEQ ID NO: 49, SEQ ID NO: 58, SEQ ID NO: 66 and SEQ ID NO: 79, SEQ ID NO: 25, SEQ ID NO: 29, SEQ ID NO: 30, SEQ ID NO: 59 and SEQ ID NO: 69, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, SEQ ID NO: 12, SEQ ID NO: 34, SEQ ID NO: 50, SEQ ID NO: 60, SEQ ID NO: 62, SEQ ID NO: 64 and SEQ ID NO: 74, with a reference quantity from a sample obtained from a subject suffering from arthrosis, and
d) assigning the subject of step (a) to the group of subjects suffering from psoriatic arthritis when they present:
   (i) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 22, SEQ ID NO: 26, SEQ ID NO: 27, SEQ ID NO: 31, SEQ ID NO: 37, SEQ ID NO: 39, SEQ ID NO: 46, SEQ ID NO: 47, SEQ ID NO: 48, SEQ ID NO: 49, SEQ ID NO: 58, SEQ ID NO: 66 and SEQ ID NO: 79, detected in (b); or
   (ii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and SEQ ID NO: 11, and a significantly lower expression level and/or quantity of the peptides with the sequences SEQ ID NO: 25, SEQ ID NO: 29, SEQ ID NO: 30, SEQ ID NO: 59 and SEQ ID NO: 69, detected in (b); or
   (iii) a significantly higher expression level and/or quantity of the peptides of step (i), and the peptides with the sequences SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, detected in (b); or
   (iv) a significantly higher expression level and/or quantity of the peptides of step (i), and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, detected in (b); or
   (v) a significantly higher expression level and/or quantity of the peptides of step (iii), and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, detected in (b); or
   (vi) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, and a significantly lower expression level and/or quantity of the peptides with the sequences SEQ ID NO: 25, SEQ ID NO: 29, SEQ ID NO: 30, SEQ ID NO: 59 and SEQ ID NO: 69, detected in (b); or
   (vii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and SEQ ID NO: 11, and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, SEQ ID NO: 25, SEQ ID NO: 29, SEQ ID NO: 30, SEQ ID NO: 59 and SEQ ID NO: 69, detected in (b); or
   (viii) a significantly higher expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77 and SEQ ID NO: 78, and a significantly lower expression level and/or quantity of the peptides with the sequences SEQ ID NO: 12, SEQ ID NO: 34, SEQ ID NO: 50, SEQ ID NO: 60, SEQ ID NO: 62, SEQ ID NO: 64 and SEQ ID NO: 74, SEQ ID NO: 25, SEQ ID NO: 29, SEQ ID NO: 30, SEQ ID NO: 59 and SEQ ID NO: 69, detected in (b);
as compared to the expression level and/or the quantity of the same peptides detected in a reference sample from a group of subjects suffering from arthrosis.

The reference quantity is obtained from the expression values and/or constitutive quantities of the peptide(s) described in the present disclosure for a group of healthy subjects or individuals, or, preferably, who do not present an arthrotic disease, or from a group of subjects who present an arthrotic disease, according to each of the methods described above and throughout the present document. The reference sample may be analysed, for example, simultaneously or consecutively with the test biological sample. The comparison described in the methods of the present invention or of the present disclosure may be performed manually or with computer assistance.

In a more preferred aspect of all the methods described in the present document, these are characterized in that steps (b) and/or (c) may be totally or partially automated, for example, by means of a sensor robotic equipment, for the detection of the quantity of step (b) or the computerised comparison of step (c).

In another more preferred aspect of all the methods described in the present document, these are characterized in that the sample is a biological fluid.

In the present disclosure, "diagnosis" is understood as the process whereby a given disease, nosological entity, syndrome, or any health-disease condition is identified, by analysing a number of clinical parameters or symptoms characteristic of said disease, and which differentiate it from other diseases with similar symptoms. In the present disclosure, the disease, nosological entity or syndrome to be detected is arthrosis, and the clinical parameter is the peptide pattern of the disclosure.

In the present disclosure, "rheumatic diseases" are understood as those disorders that generally affect the locomotor and the musculo-skeletal system, preferably the joints, tendons, ligaments, bones and muscles. For the purposes of the present disclosure, the rheumatic diseases are selected from any of the following: arthrosis, rheumatoid arthritis and psoriatic arthritis.

For the purposes of the present disclosure, the term "arthrosis" or "osteoarthritis" refers to a chronic articular disease caused by deterioration of the hyaline cartilage and osteoblastic hyperactivity of subchondral bone. In the present disclosure, the terms "arthrosis" and "osteoarthritis" are equivalent and may be used interchangeably throughout the description thereof. As known to persons skilled in the art, arthrosis may be classified into idiopathic arthrosis and secondary arthrosis (modified from Altman RC, Semin Arthritis Rheum 1991; 20:40-47). Idiopathic arthrosis (the origin of the disease is unknown) may be localised, for example in the hands, feet, hips, spine, etc., or generalised, in which case three or more articular areas are affected, such as the small joints and the spine, the large joints and the spine, or mixed, which is a combination of the above. Secondary arthrosis is classified into congenital or developmental diseases (including, without being limited thereto, Gaucher's disease, the Legg-Calvé disease, bone dysplasias, etc.), calcium deposition diseases (which include, without being limited thereto, hydroxyapatite arthropathy, destructive arthropathy and calcium pyrophospate deposition arthropathy), post-traumatic diseases, bone and articular diseases (which include, without being limited thereto, avascular necrosis, rheumatoid arthritis, gouty arthritis, septic arthritis, Paget's disease, osteopetrosis, osteochondritis), and various diseases originating from arthritis (which include, without being limited thereto, Charcot joint, diabetes mellitus, acromegaly, hypothyroidism, hyperparathyroidism, Kashin-Beck disease, Caisson's disease, etc.). On the other hand, arthrosis may also be classified using the radiographic KELLGREN-LAWRENCE (K/L) scale, which classifies arthrosis into 5 radiographic grades: Grades 0, I, II, III, and IV.

Any of the diseases included within the classification of arthrotic diseases, including arthrosis, rheumatoid arthritis and psoriatic arthritis, may be diagnosed and classified using the peptide pattern of the disclosure.

For purposes of the present disclosure, the term "rheumatoid arthritis (RA)" refers to an inflammatory disease of unknown etiology, characterized by affectation of the diarthrodial joints and the development of chronic synovitis, which progressively leads to articular damage, functional impairment and a significant deterioration of the patients' quality of life. In addition to articular affectation, RA is also characterized by a sustained systemic inflammatory process and the possibility of developing severe extra-articular manifestations, which often determine the prognosis of the disease.

For purposes of the present disclosure, the term "psoriatic arthritis (PsA)" refers to an articular disease that appears in some patients (approximately 10%) who suffer from psoriasis of the skin, which gives it peculiar characteristics in terms of evolution and prognosis. The articular injury is inflammatory, i.e. is accompanied by pain, swelling, warmth, difficulty moving the inflamed joint and, in the long term, the possibility of deformation. It is a chronic disease, which evolves in an irregular manner throughout life, with periods of inactivity and periods of inflammation and pain.

In the present disclosure, "subject" or "individual" is understood to mean any animal, preferably a mammal, more preferably a primate, and, in particular, a human being, of any race, sex, age or physical condition. However, in a particular aspect, the subject is a woman; more particularly, a woman older than 50.

The implementation of the methods of the disclosure described herein comprises determining the expression level and/or the concentration or quantity of the peptides of the disclosure; preferably, it comprises determining the presence or absence of the peptides that comprise the peptide pattern of the disclosure in a biological sample from said subject or individual.

In the present disclosure, "biological sample" is understood to mean any material of animal origin, in particular of human origin, including excreta (faeces and urine), secretions (genital, respiratory, etc.), blood or the components thereof, organic tissues and fluids (biopsies, fluids: cephalo-rachidian, synovial, articular, ascitic, etc.), etc. In a particular aspect of the methods of the disclosure, the sample taken from the subject is a biological fluid, which, in another, even more particular aspect, is selected from the group consisting of blood, serum, plasma, synovial fluid and urine; more preferably, serum, plasma and synovial fluid.

Once the biological sample has been obtained from the subject, it must be processed to obtain the peptides described in the present disclosure. Thus, the biological sample may be treated such that the structure of the tissue or cell is physically or mechanically broken down and releases the intracellular components into an aqueous or organic solution. Once this has been performed, the proteins are isolated from the sample. Any of the techniques known in the state of the art to isolate proteins may be used in the present disclosure, such as, for example, centrifugation, depletion, etc. Once the proteins have been isolated, they are digested in order to obtain the peptide profile of the disclosure.

Once the peptide fraction has been obtained, and depending on the technique used to analyse the expression, the protein fraction obtained may or may not be digested with trypsin, to perform enzymatic digestion of the proteins and obtain peptides. Trypsin is a peptidase enzyme that breaks down the peptide bonds within proteins by means of hydrolysis, to form smaller-size peptides and amino acids. Trypsin is produced in the pancreas and secreted in the duodenum (part of the intestine), where it is essential for digestion. The optimal pH is 8 and the optimal temperature is 37°C. For purposes of the present disclosure, any technique known and used in the state of the art to isolate the peptide fraction from a biological sample may be used in the present disclosure.

In another more preferred aspect of the disclosure, the methods described herein are characterized in that the detection of the expression levels and/or the quantity or concentration of the peptides is performed by means of techniques that are widely known in the state of the art. Examples of said techniques include, without being limited thereto, mass spectrometry, gas chromatography-mass spectrometry or liquid chromatography-mass spectrometry, flow cytometry, Western Blot or Western transfer, ELISA (enzyme-linked immunosorbent assay), RIA (radioimmunoassay), competitive EIA (competitive enzyme immunoassay), DAS-ELISA (double-antibody sandwich ELISA) and immunocytochemical and immunohistochemical techniques.

Mass spectrometry is an analytical technique that makes it possible to elucidate chemical structures by measuring the mass/charge ratio of molecular species. The determinations require generating electrically charged species, which is performed by means of different methodologies, such as electron impact, fast atom bombardment (FAB) and generation of bound ions. The measurement of the mass/charge ratio makes it possible to determine the exact molecular weight of the molecule. Other techniques derived from mass spectrometry that may also be used within the context of the present disclosure include, without being limited thereto, gas chromatography-mass spectrometry (GC-MS) and liquid chromatography-mass spectrometry (LC-MS).

The ELISA technique (acronym for Enzyme-Linked ImmunoSorbent Assay) is an immunoassay technique wherein an immobilised antigen is detected by means of an enzyme-linked antibody capable of generating a detectable product, such as a change in colour or any other type.

In the present disclosure, the term "antibody" is broadly interpreted to include polyclonal, monoclonal and multi-specific antibodies, and fragments thereof [F(ab')2, Fab, scFv, etc.], provided that they are capable of recognising the antigen of interest, i.e. of binding to the peptides comprised within the peptide pattern of the disclosure. Examples of antibodies that may be used within the context of the present disclosure include, without being limited thereto, polyclonal antibodies, monoclonal antibodies, recombinant antibodies, chimeric antibodies, humanised antibodies, totally human antibodies, etc. The antibodies used in these assays may or may not be labelled. Illustrative examples of labels that may be used include radioactive isotopes, enzymes, fluorophores, chemiluminescent reagents, enzyme substrates or co-factors, enzyme inhibitors, particles, dyes, etc.

Flow cytometry is a biophysical technology based on the use of laser light, wherein the proteins, which are bound to a labelled antibody and suspended in a fluid, go through a very thin transparent tube that is hit by a thin laser beam; the light transmitted and dispersed when the proteins go through the tube is collected by means of detection devices, making it possible to detect and separate the proteins bound to the labelled antibodies.

The detection of the quantity of the peptides described throughout the present document may be performed by any means known in the state of the art. Thus, the detection of the levels or quantities of said peptides may be performed in a semi-quantitative or quantitative manner, which makes it possible to differentiate between the different types of subjects, healthy subjects and those suffering from arthrosis, rheumatoid arthritis or psoriatic arthritis. In this way, a differential diagnosis may be made for the subjects affected by the aforementioned diseases, which makes it possible to sub-classify them.

The measurement of the quantity or concentration of these peptides, preferably in a semi-quantitative or quantitative manner, may be performed directly or indirectly. Direct measurement refers to measuring the quantity or concentration of the expression product thereof, based on a signal that is directly obtained from the expression of said peptides, and is directed correlated to the number of proteins present in the samples analysed. Said signal - which may also be called intensity signal - may be obtained, for example, by measuring the intensity value of a chemical or physical property of said products. Indirect measurement includes the measurements obtained from a secondary component or a biological measurement system (for example, measurement of cellular responses, ligands, "labels" or enzymatic reaction products).

As used in the description, the term "quantity" refers, without being limited thereto, to the absolute or relative quantity of the peptides or the molecules capable of detecting them, as well as to any other value or parameter related to them or which may be derived from them. Said values or parameters comprise signal intensity values obtained for any physical or chemical property of said expression products by means of direct measurement. Moreover, said values or parameters include all those obtained by means of indirect measurement, for example, any of the measurement systems described elsewhere in the present document.

Another aspect of the disclosure relates to a kit or device, hereinafter "kit of the disclosure", which comprises the necessary molecular tools to detect the expression level and/or quantity of the peptides described in the present disclosure, or any combination thereof, for the diagnosis and classification of rheumatic diseases.

In a more preferred aspect, the kit of the disclosure comprises the necessary molecular tools to detect the expression level and/or quantity of the peptides with the sequences SEQ ID NO: 1 to 11.

In another, even more preferred aspect, the kit of the disclosure further comprises the necessary molecular tools to detect the expression level and/or quantity of at least one of the peptides with the sequences: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 61, SEQ ID NO: 62, SEQ ID NO: 64, SEQ ID NO: 65, SEQ ID NO: 66, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 69, SEQ ID NO: 79, or combinations thereof.

In a more preferred aspect of the kit, said molecules capable of detecting the expression levels and/or quantities of the peptides described in the present disclosure are preferably selected from any of the following: specific antibodies against the peptides that comprise the peptide pattern of the disclosure or fragments thereof, and oligonucleotides capable of hybridising to the nucleotide sequence that encodes the peptides described in the present disclosure.

In a preferred aspect, said oligonucleotides are designed from a nucleotide sequence that encodes the peptides described in the present document.

In a preferred aspect, the kit of the disclosure is useful for the diagnosis and classification of rheumatic diseases in a subject or individual, wherein said rheumatic diseases are selected from any of the following: arthrosis, rheumatoid arthritis and psoriatic arthritis.

The terms "peptide pattern", "peptides" and "antibody" have already been described and explained in the preceding section, which is applicable to the present aspect.

In another preferred aspect, the kit of the disclosure comprises secondary antibodies or positive and/or negative controls. Moreover, the kit may include, without any type of limitation, buffers, protein extraction solutions, contamination prevention agents, protein degradation inhibitors, etc. On the other hand, the kit may include all the necessary supports and containers for the implementation and optimisation thereof. Preferably, the kit further comprises instructions for performing the methods of the disclosure.

Within the context of the present disclosure, "kit" is understood to mean a product that contains the different reagents needed to implement the method of the disclosure, packed in order to allow for the transport and storage thereof. Suitable materials for packing the kit components include, without being limited thereto, glass, plastic (polyethylene, polypropylene, polycarbonate and similar materials), bottles, vials, paper, sachets and similar materials.

Furthermore, the kit may contain instructions for using the different kit components. Said instructions may be in the form of printed material or in the form of an electronic medium capable of storing instructions such that they may be read by a subject, such as electronic storage media (magnetic disks, tapes and similar media), optical media (CD-ROM, DVD) and similar media. Additionally or alternatively, the media may contain Internet addresses that supply said instructions.

Another aspect of the present disclosure relates to the use of the kit of the disclosure to obtain useful data for the diagnosis and classification of rheumatic diseases, preferably arthrosis, rheumatoid arthritis and psoriatic arthritis, from a biological sample. In a more preferred aspect, the biological sample is a biological fluid, which is preferably selected from the group consisting of blood, serum, plasma, synovial fluid and urine.

Throughout the description and the claims, the word "comprises" and variants thereof are not intended to exclude other technical characteristics, additives, components or steps. For persons skilled in the art, other objects, advantages and characteristics of the disclosure will arise, partly from the description and partly from the implementation of the disclosure.

### BRIEF DESCRIPTION OF THE FIGURES

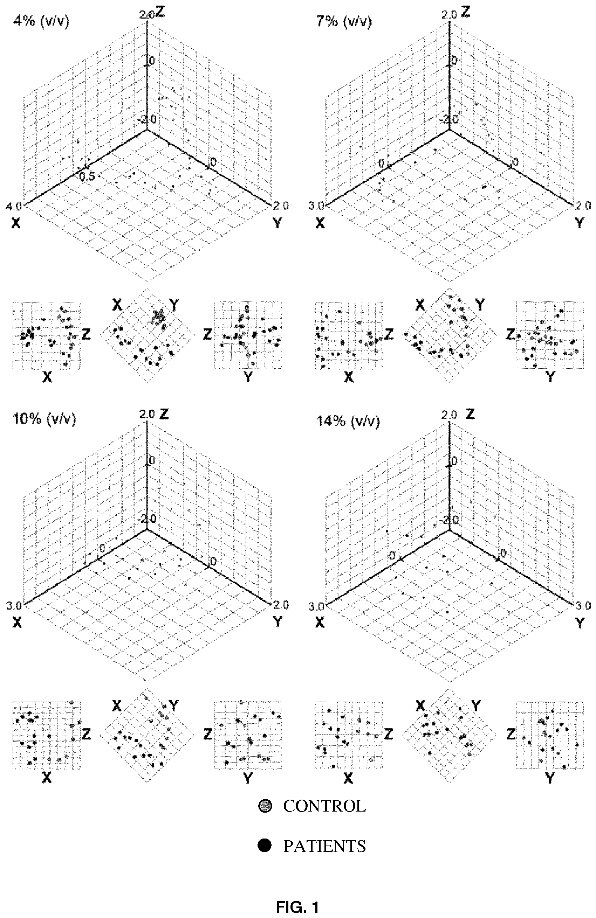

**Figure 1****:** Diagrams of the principal component analyses (PCA) performed with the peptides of the disclosure and obtained by means of the MALDI-TOF technique, for the group of arthrotic patients (grey) and for the group of control patients (black), at each elution percentage. The PCAs show that, under all the extraction conditions, classification of the samples is achieved in at least two planes.

### EXAMPLES

The following examples and figures are provided for illustrative purposes, and are not intended to limit the scope of the present invention.

### Example 1. MATERIAL AND METHODS

### 1.1 Reagents

DL-dithiothreitol (DTT ≥ 99%)
lodoacetamide (IAA ≥ 99%)
Trifluoroacetic acid (TFA ≥ 99%, for LC-MS)
Acetonitrile (ACN, LC-MS CHROMASOLV)
Water (LC-MS CHROMASOLV)
Ammonium bicarbonate (≥ 99 %)
Trypsin (sequencing grade, Roche)
Large NuTips, 10-200 µl, C-18 (Glygen, Columbia, USA)
α-Cyano-4-hydroxycinnamic acid, ultra-pure, for MALDI-MS (Fluka, Germany)
Calibration mixture 1,4700 Proteomics Analyzer (ABSciex, Framingham, MA, USA)

### 1.2. Serum samples

The human serum samples were obtained from anonymous donors at the A Coruña University Hospital Complex, Spain. All the patients signed the informed consent. The study was approved by the Local Ethics Committee of Galicia (Spain). The following were selected: 40 serum samples from arthrotic donors (OA), 40 serum samples from donors diagnosed with rheumatoid arthritis (RA), 40 samples from donors diagnosed with psoriatic arthritis (PsA), and 40 serum samples from donors without any of the aforementioned pathologies, i.e. subjects who did not present any type of rheumatic pathology. The latter group is the control group (N). 20 sera from each group of patients were used to search for the peptide pattern characteristic of each pathology. Subsequently, the other remaining 20 sera from each group of patients were used as blind samples in order to verify the validity of the peptide pattern of the invention. The serum samples were stored at -80°C until the processing thereof.

### 1.3. Sequential serum protein depletion

Protein depletion was performed according to the protocol that was previously developed at our laboratory (Fernández-Costa et al., Proteome Sci 2012; 55). Each of the sera were subjected to a sequential depletion protocol involving two precipitation steps: first with DTT and, subsequently, with ACN, according to the protocol described by Kay et al. (Rapid Commun Mass Spectrom, 2008, 22: 3255-60), with slight modifications. Briefly, 2.2 µl of 500 mM DTT were added to 20 µl of serum and vortex-stirred. Thereafter, the sample was incubated for 1 hour until a persistent white viscous precipitate was formed, which was sedimented by centrifugation at 14,000 xg for 2 x 20 minutes. The supernatant was transferred to a clean tube (LoBind, Eppendorf) and depleted once again by the addition of 57% (v/v) acetonitrile, followed by two short vortex cycles and 10 minutes of ultrasound in an ultrasonic bath (SONOREX, Bandelin). The protein precipitate was sedimented by centrifugation at 14,000 xg for 10 minutes, and the supernatant was transferred to a clean LoBind tube and evaporated to dryness using a vacuum centrifuge.

### 1.4. In-solution digestion

The ultrasonic in-solution digestion was performed according to the previously developed ultra-rapid proteolytic digestion protocol (HM Santos et al. J Proteome Res 2007.6: 3393-9). The evaporated sample was re-suspended in 20 µl of 25mM ammonium bicarbonate (AmBi), and 10 µl of acetonitrile were added and mixed by means of vortex-mixing and sonication for 1 minute (50% amplitude) in an ultrasonic bath (SONOPULS HD 2200 with BB6 accessory, Bandelin). The protein cysteine residues were reduced with 2 µl of 110 mM DTT, followed by vortex-mixing and 1 minute of sonication, and, subsequently, blocked with 2 µl of 600 mM IAA, vortex-mixing and 1 minute of sonication. In order to de-activate the IAA, 10 µl of 110 mM DTT were added to the sample. Thereafter, the sample was diluted to a final volume of 200 µl with 12.5 mM AmBi, and trypsin was added at a 1:20 (w/w) trypsin:protein ratio. The digestion was performed in the ultrasonic equipment for 5 minutes with a 50% amplitude. Finally, 2 µl of 50% (v/v) formic acid were added in order to stop the enzymatic reaction. The digested serum was evaporated to dryness in a vacuum centrifuge.

### 1.5. Sequential elution by means of NuTips (peptide separation)

The digested samples were reconstituted in 30 µl of 0.1% (v/v) TFA, and 20 µg of the digest were loaded onto a NuTip C18 (large) type tip. The peptide separation was performed according to the following protocol:
Conditioning: 30 µl of a solution of 60%-80% (v/v) ACN and 0.1% (v/v) TFA were aspirated and expelled 5 times, and washed 3 times with 0.1% TFA.
Binding: the sample was aspirated and expelled 50 times, in order to allow for the peptides to adsorb to the reversed-phase material packed in the tip.
Sequential elution: 30 µl of different percentages of ACN, from low to high concentrations of ACN, were aspirated and expelled (20 times each). The sequential elution was performed with 4%, 7%, 10% and 14% (v/v) ACN. The eluates were evaporated to dryness.

### 1.6. Analysis by means of mass spectrometry

The evaporated samples were re-suspended in 6 µl of 0.1% (v/v) TFA, and 1 µl of each sample was deposited in quintuplicate on a MALDI plate (384-well plate coated with Teflon®), and allowed to air-dry at room temperature. Subsequently, 1 µl of a solution of 3 mg/ml of α-cyano-4-hydroxycinnamic acid in 0.1% (v/v) TFA and 50% (v/v) ACN was added to the dry peptide samples, and allowed to dry once again.

The samples were analysed in positive-ion mode using a 4800 Proteomics Analyzer MALDI-TOF/TOF mass spectrometer (ABSciex, Framingham, MA, USA), equipped with an Nd:YAG laser (λ = 355 nm) and the 4000 Series Explorer™ programme (ABSciex). The internal calibration, data acquisition, processing and interpretation were performed following the manufacturer's recommendations. All the mass spectra must be externally calibrated using a standard peptide mixture (ABSciex). The mass data were exported on the basis of the following characteristics: a) mass range: 500-4000 (Da); b) density of peaks: maximum 50 peaks per 200 Da; c) signal-to-noise ratio: minimum 20; d) minimum surface area: 100; e) maximum peaks /spot: 500.

The analysis of the mass spectrum for each sample corresponds to an average 1,500 laser pulses. The fragmentation spectra were acquired by selecting the precursors of interest in each MALDI-TOF mass map, with an average 2,000 laser pulses per spectrum. For the fragmentation (MS/MS), all the peaks with a signal-to-noise ratio (S/N) > 10 were included in order to perform the database search.

The peptide sequencing was performed using the ProteinPilot™ software (ABSciex), with the following parameters: (i) Version UniProt: 2013_09, which contains 540,958 sequence entries, (ii) Taxonomy: Homo sapiens, (iii) Enzyme: trypsin or without enzyme; (v) Fixed modifications: iodoacetamide. Only those peptides identified with at least 70% confidence were reported.

### 1.7. Bio-computer analysis

Each spectrum was pre-processed using the following parameters: mass range between 500 and 4000 Da, maximum density of peaks: 10 peaks per 200 Da, minimum signal-to-noise ratio (S/N) of 10, minimum surface area of 100, maximum number of peaks per spectrum: 120. The peaks were aligned with a peptide mass tolerance of 150 ppm and, finally, a representative spectrum was created for each sample with peaks present in at least 4 of the 5 replica spectra of the sample. For each peak, only their presence or absence in each spectrum was taken into consideration, the use of their intensity beyond pre-processing being discarded.

In order to identify the peaks that were differentially expressed in the different groups, Fisher's test of independence was applied for comparisons between two groups and the Chi-square test of independence was applied for comparisons between the four groups analysed. Since these tests were performed for all the peaks detected, the Benjamini-Hochberg False Discovery Rate (FDR) correction was subsequently applied, in order to reduce the number of false positives.

The separation between the samples under different conditions was graphically analysed using a Principal Component Analysis (PCA).

Finally, in order to verify the utility of the peptides that comprise the peptide pattern described in the present invention for use in the diagnosis of arthritis, the performance of automatic classifiers trained only with the information from these peptides was evaluated on a set of blind samples.

### Example 2. RESULTS

### 2.1. Obtainment of the peptide pattern characteristic of arthrosis

In order to obtain the peptide pattern described in the present invention, 20 serum samples from each of the groups of patients included in the present study (OA: arthrosis, RA: rheumatoid arthritis, PsA: psoriatic arthritis, and N: control) were used and, by means of the techniques described in Example 1, the peptides characteristic of each of the study groups that were analysed were detected. Briefly: in first place, the peptides that were significantly detected in one pathology as compared to the others, on the basis of a corrected q value < 0.05 for Fisher's test for pairwise comparisons, were selected. Subsequently, those peptides that were significantly detected under two or more conditions on the basis of a corrected q value < 0.05 for the Chi-square test of independence, as indicated above, were selected.

By means of said methodology, a total of 11 peptides were identified which were significantly expressed in the group of arthrotic patients as compared to the rest of the groups included in the study. This specific peptide pattern makes it possible to distinguish the samples from those subjects with arthrosis (OA) from the samples from the control subjects (N), and even from the samples of those subjects who suffer from other rheumatic diseases, such as psoriatic arthritis (PsA) and rheumatoid arthritis (RA).

Said set of 11 peptides that make up the peptide pattern of the invention are shown in **Table 7.**

**Table 7: Masses of the characteristic peptides that comprise the peptide pattern for the diagnosis of patients suffering from arthrosis (OA). The percentages indicate the presence of each peptide in the total samples analysed for each group of patients, PsA: group of patients suffering from psoriatic arthritis; RA: group of patients suffering from rheumatoid arthritis; N: group of control subjects.**

| **m/z** | **SEQ ID No.** | **PsA (n = 20)** | **RA (n = 20)** | **OA (n = 20)** | **N (n = 20)** |
|---|---|---|---|---|---|
| **1392.7517** | 1 | 0% | 5% | 50% | 10% |
| **1532.796** | 2 | 5% | 15% | 40% | 0% |
| **1758.979** | 3 | 5% | 5% | 40% | 0% |
| **1909.9498** | 4 | 0% | 5% | 40% | 0% |
| **2165.146** | 5 | 10% | 5% | 45% | 0% |
| **2180.1414** | 6 | 15% | 10% | 70% | 20% |
| **2280.1501** | 7 | 5% | 5% | 40% | 5% |
| **2291.227** | 8 | 5% | 10% | 60% | 20% |
| **2407.3042** | 9 | 5% | 0% | 45% | 0% |
| **2436.3015** | 10 | 35% | 25% | 90% | 40% |
| **2663.4382** | 11 | 5% | 5% | 60% | 15% |

The peptides selected as characteristic of arthrosis appear with a significantly greater frequency in the sera of arthrotic donors as compared to the sera of the control patients or patients suffering from PsA or RA.

### 2.2. Additional specific peptides for the diagnosis of other pathologies, such as RA and PsA

In addition to detecting the peptide pattern described in the invention, by means of the methods described above, a set of peptides, in addition to the 11 peptides that comprise the peptide pattern of the invention for use in the diagnosis of arthrosis, was selected, in order to improve classification of the samples, preferably serum samples, from arthrotic donors. Said additional peptides were selected from the data obtained from the analysis of the 80 serum samples analysed. In this case, since there were comparisons between pairs of conditions, the peptides selected were those for which a corrected q value < 0.05 was obtained for Fisher's test.

**Table 8** shows the additional peptides that may be used together with the peptide pattern of the invention and which are capable of differentiating between arthrotic subjects and normal subjects who do not suffer from any arthrotic pathology.

**Table 8. Masses of the additional peptides, other than the peptide pattern of the invention, useful for classifying and diagnosing patients suffering from arthrosis (OA), and distinguishing them from subjects who do not suffer from any arthrotic pathology. The percentages indicate the presence of each peptide in the total samples analysed for each group of patients.**

| **m/z** | **SEQ ID No.** | **OA (n = 20)** | **N (n = 20)** |
|---|---|---|---|
| **896.490** | **13** | **65%** | **5%** |
| **961.627** | **14** | **70%** | **15%** |
| **980.529** | **15** | **70%** | **20%** |
| **1019.604** | **16** | **65%** | **10%** |
| **1108.588** | **17** | **50%** | **0%** |
| **1117.669** | **18** | **55%** | **5%** |
| **1148.634** | **19** | **75%** | **15%** |
| **1213.667** | **20** | **60%** | **5%** |
| **1237.674** | **21** | **50%** | **0%** |
| **1255.716** | **24** | **50%** | **0%** |
| **1403.749** | **28** | **45%** | **0%** |
| **1470.813** | **32** | **70%** | **5%** |
| **1502.810** | **33** | **65%** | **5%** |
| **1568.914** | **36** | **75%** | **20%** |
| **1585.846** | **38** | **50%** | **10%** |
| **1641.892** | **40** | **85%** | **35%** |
| **1641.892** | **41** | **85%** | **35%** |
| **1669.862** | **42** | **80%** | **45%** |
| **1706.931** | **43** | **70%** | **5%** |
| **1815.922** | **51** | **60%** | **10%** |
| **1825.005** | **52** | **80%** | **30%** |
| **1834.946** | **53** | **45%** | **5%** |
| **1864.027** | **54** | **75%** | **25%** |
| **1875.997** | **55** | **75%** | **20%** |
| **1876.017** | **56** | **75%** | **20%** |
| **1884.000** | **57** | **65%** | **20%** |
| **2186.092** | **65** | **60%** | **10%** |
| **2296.219** | **67** | **80%** | **20%** |
| **2315.195** | **68** | **75%** | **30%** |
| **2348.245** | **70** | **95%** | **30%** |
| **2652.448** | **75** | **70%** | **5%** |
| **2670.387** | **76** | **50%** | **15%** |
| **2695.362** | **77** | **95%** | **40%** |
| **2790.425** | **78** | **55%** | **15%** |

Alternatively, **Table 9** shows the additional peptides that may be used together with the peptide pattern of the invention and which are also capable of differentiating between arthrotic subjects and normal subjects who do not suffer from any arthrotic pathology. The difference between the data shown in Table 8 and Table 9 lies in the fact that, in Table 8, arthrotic subjects show a higher presence of said peptides, whereas the data in Table 9 show that arthrotic subjects have a lower presence of said peptides as compared to the healthy control subjects.

**Table 9. Masses of the additional peptides, other than the peptide pattern of the invention, useful for classifying and diagnosing patients suffering from arthrosis (OA), and distinguishing them from subjects who do not suffer from any arthrotic pathology. The percentages indicate the presence of each peptide in the total samples analysed for each group of patients.**

| **m/z** | **SEQ ID No.** | **OA (n = 20)** | **N (n** = **20)** |
|---|---|---|---|
| **871.502** | **12** | **0%** | **30%** |
| **1518.710** | **34** | **15%** | **55%** |
| **1810.992** | **50** | **5%** | **40%** |
| **1922.877** | **60** | **5%** | **50%** |
| **2012.011** | **62** | **40%** | **80%** |
| **2153.112** | **64** | **30%** | **90%** |
| **2602.358** | **74** | **35%** | **65%** |

**Table 10** shows the additional peptides that may be used jointly with the peptide pattern of the invention and which are capable of differentiating between arthrotic subjects and subjects who suffer from rheumatoid arthritis. It is worth noting that, as may observed in said Table 10, subjects suffering from OA present a higher presence of the peptides with the sequences SEQ ID NO: 30, 59 and 69, as compared to those patients who suffer from RA. On the contrary, arthrotic subjects present a lower presence of the peptides with the sequences SEQ ID NO: 22, 23, 26, 31, 35, 39, 45, 46, 47, 48, 58, 61, 63, 66, 71, 72 and 73, as compared to those subjects who suffer from RA.

**Table 10. Masses of the additional peptides, other than the peptide pattern of the invention, useful for classifying and diagnosing patients suffering from arthrosis (OA) and distinguishing them from subjects who suffer from rheumatoid arthritis (RA). The percentages indicate the presence of each peptide in the total samples analysed for each group of patients.**

| **m/z** | **SEQ ID No.** | **RA (n = 20)** | **OA (n = 20)** |
|---|---|---|---|
| **1247.652** | **22** | **50%** | **25%** |
| **1249.655** | **23** | **30%** | **0%** |
| **1299.609** | **26** | **90%** | **20%** |
| **1465.689** | **30** | **30%** | **80%** |
| **1467.852** | **31** | **90%** | **45%** |
| **1519.750** | **35** | **30%** | **0%** |
| **1612.849** | **39** | **55%** | **20%** |
| **1736.970** | **45** | **35%** | **0%** |
| **1743.882** | **46** | **45%** | **10%** |
| **1745.901** | **47** | **45%** | **5%** |
| **1771.885** | **48** | **90%** | **25%** |
| **1899.060** | **58** | **55%** | **15%** |
| **1906.873** | **59** | **5%** | **80%** |
| **1932.038** | **61** | **45%** | **0%** |
| **2020.138** | **63** | **60%** | **15%** |
| **2190.112** | **66** | **85%** | **20%** |
| **2344.169** | **69** | **5%** | **80%** |
| **2394.298** | **71** | **60%** | **10%** |
| **2409.281** | **72** | **50%** | **0%** |
| **2565.322** | **73** | **70%** | **20%** |

**Table 11** shows the additional peptides that may be used together with the peptide pattern of the invention and which are capable of differentiating between arthrotic subjects and subjects who suffer from psoriatic arthritis. It is worth noting that, as may be observed in said Table 11, subjects suffering from OA present a higher presence of the peptides with the sequences SEQ ID NO: 25, 29, 30, 59 and 69, as compared to those patients who suffer from PsA. On the contrary, arthrotic subjects present a lower presence of the peptides with the sequences SEQ ID NO: 22, 26, 27, 31, 37, 39, 46, 47, 48, 49, 58, 66 and 79, as compared to those subjects who suffer from PsA.

**Table 11. Masses of the additional peptides, other than the peptide pattern of the invention, useful for classifying and diagnosing patients suffering from arthrosis (OA) and distinguishing them from subjects who suffer from psoriatic arthritis (PsA). The percentages indicate the presence of each peptide in the total samples analysed for each group of patients.**

| **m/z** | **SEQ ID No.** | **PsA(n = 20)** | **OA (n = 20)** |
|---|---|---|---|
| **1247.652** | **22** | **55%** | **25%** |
| **1263.683** | **25** | **10%** | **60%** |
| **1299.609** | **26** | **90%** | **20%** |
| **1380.780** | **27** | **30%** | **0%** |
| **1449.800** | **29** | **25%** | **75%** |
| **1465.689** | **30** | **5%** | **80%** |
| **1467.852** | **31** | **95%** | **45%** |
| **1569.907** | **37** | **50%** | **0%** |
| **1612.849** | **39** | **75%** | **20%** |
| **1743.882** | **46** | **55%** | **10%** |
| **1745.901** | **47** | **25%** | **5%** |
| **1771.885** | **48** | **95%** | **25%** |
| **1786.909** | **49** | **65%** | **20%** |
| **1899.060** | **58** | **50%** | **15%** |
| **1906.873** | **59** | **0%** | **80%** |
| **2190.132** | **66** | **70%** | **20%** |
| **2344.169** | **69** | **0%** | **80%** |
| **2940.755** | **79** | **60%** | **20%** |

### 2.3. Principal component analysis (PCA).

Using the peptides selected in the peptide profile described in the invention, obtained from the serum samples from the group of arthrotic patients and the samples from the control group, a principal component analysis (PCA) was performed, which verified that the samples are separated into at least two planes under all elution conditions, such that the peptides are capable of differentiating between samples from arthrotic donors and those from the control subjects.

Prior to performing the PCA, a single data set was created for each elution percentage (4%, 7%, 10% and 14%), with the representative spectrum for each sample. The spectra of each set of data were converted into 1s and 0s vectors, where 1 means presence of a peak and 0 means absence of a peak.

The PCA analysis and the visualisation thereof were performed using the RapidMiner v5.3 programme (http://rapid-i,com/content/view/181/190/), which configures the PCA algorithm in order to reduce the dimensionality of the data sets to 3 principal components.

These results are shown in **Figure 1****.**

### 2.4. Testing of the classifier with blind samples

The utility of the peptides that comprise the peptide pattern described in the present invention for use in the diagnosis of arthrosis was tested in a group of patients whose pathology was unknown (blind samples). The samples from said patients (n = 79) were processed in the same manner as that described above in the materials and methods section.

Using the set of data described in section 2.1, a number of automatic classifiers were evaluated using the Weka 3.7 machine learning tool (http://www.cs.waikato.ac.nz/ml/weka/). These classifiers were trained with the different possible combinations of the data from each elution. This evaluation was performed using the cross-validation scheme known as Leave-One-Out on two different problems: *i*) classification of the samples under all conditions, and *ii*) classification of those samples that present a condition related to a pathology (RA, PsA and OA groups). As a result of this evaluation, the best classifier selected for the problem of classifying all the conditions was the LibSVM implementation of the Support Vector Machine (SVM) algorithm with a Radial Basis Function (RBF) kernel trained with the data from the 7% and 10% elution percentages, combined with an optimiser based on a grid search (GridSearch algorithm) configured to search for the optimal value of the "cost" parameter amongst the values 2⁻⁵ , Z⁻³, 2⁻¹, 2¹, 2³, 2⁵, 2⁷, 2⁹, 2¹¹, 2¹³ and 2¹⁵, and the optimal value of the "gamma" parameter amongst the values 2⁻¹⁵, 2⁻¹³ 2⁻¹¹, 2⁻⁹, Z⁻⁷, Z⁻⁵, Z⁻³, 2⁻¹, 2¹ and 2³. On the other hand, the Artificial Neural Network (ANN) classifier trained with the data from the 10% percentages was selected as the best classifier for the problem of classifying the pathological conditions.

Using these classifiers trained with the set of data described in section 2.1, the set of data from the blind samples were classified. In the case of the SVM-RBF classifier for the problem of all conditions, the classifying power was only moderate, since it resulted in an accuracy ratio slightly greater than 60% and an approximate Cohen's statistic value (kappa) of 0.48. On the other hand, the ANN classifier for the problem of pathological conditions showed better results, with an accuracy ratio close to 75% and a kappa value of 0.619. The results of the classification are shown in **Table 12.**

**Table 12: Automatic classifiers created using the peptide pattern described in the present invention, obtained from the serum samples analysed for each group of patients (n = 20 for each group of patients). Conds.: conditions, Clas.: classifier, Elu.: elution percentages, %Ac.: accuracy ratio, K: global kappa, Cond.: condition, Sen.: sensitivity by condition, Sp.: specificity by condition, n: samples per condition, N: total number of samples, CD: correct diagnosis, CDP: correct diagnosis by pathology.**

| **Conds.** | **Clas.** | **Elu.** | **%Ac.** | **K** | **Cond.** | **Sen.** | **Sp.** | **n** | **N** | **CD** | **CDP** |
|---|---|---|---|---|---|---|---|---|---|---|---|
| All | SVM-RBF-OPT | 7% | 60.76 | 0.477 | PsA | 0.80 | 0.93 | 20 | 79 | 48 | 16 |
| | | | | | RA | 1.00 | 0.92 | 19 | | | 19 |
| | | 10% | | | OA | 0.45 | 0.75 | 20 | | | 9 |
| | | | | | N | 0.20 | 0.88 | 20 | | | 4 |
| **Pathologies** | ANN | 10% | 74.58 | 0.619 | PsA | 0.55 | 0.90 | 20 | 59 | 44 | 11 |
| | | | | | RA | 0.89 | 0.38 | 19 | | | 17 |
| | | | | | OA | 0.80 | 0.23 | 20 | | | 16 |

## Claims

1. Simultaneous *in vitro* use of the expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, as a marker for the diagnosis of arthrosis in a biological sample isolated from a subject.

2. *In vitro* use according to claim 1, further comprising the expression level and/or quantity of at least one of the peptides selected from the list consisting of: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof.

3. *In vitro* use according to any of claims 1 to 2, wherein the isolated biological sample is a biological fluid, preferably selected from the group consisting of blood, serum, plasma, synovial fluid and urine.

4. Method *in vitro* for the diagnosis of arthrosis in an isolated sample of a subject, which comprises:
(a) simultaneously detecting, in the isolated biological sample of the subject, the expression level and/or quantity of the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, and
(b) comparing the expression level and/or quantity obtained in step (a) with a reference quantity from an isolated biological sample obtained from a healthy control subject,
wherein a significantly higher expression level and/or quantity of the peptides in the isolated biological sample from the subject of step (a) with respect to the expression level and/or quantity of the peptides in the isolated biological sample from the healthy control subject, is indicative of arthrosis.

5. Method according to claim 4, further comprising determining, in step (a), the expression level and/or quantity of at least one of the peptides selected from the list consisting of: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, or combinations thereof,
wherein a significantly higher expression level and/or quantity of at least one of the peptides selected from the list consisting of: SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77, SEQ ID NO: 78, or any combinations thereof, and/or a significantly lower expression level and/or quantity of at least one of the peptides selected from the list consisting of: SEQ ID NO: 12, SEQ ID NO: 34, SEQ ID NO: 50, SEQ ID NO: 60, SEQ ID NO: 62, SEQ ID NO: 64, SEQ ID NO: 74, or any combinations thereof, in the isolated biological sample from the subject of step (a) with respect to the expression level and/or quantity of the peptides in the isolated biological sample from the healthy control subject, is indicative of arthrosis.

6. Method *in vitro* according to any of claims 4 to 5, wherein the isolated biological sample is a biological fluid, preferably selected from the group consisting of blood, serum, plasma, synovial fluid and urine.

7. Method *in vitro* according to any of claims 4 to 6, wherein the detection of the expression levels and/or quantity of the peptides is performed by means of mass spectrometry, ELISA, array, microarray, flow cytometry, mass spectrometry, gas chromatography-mass spectrometry or liquid chromatography-mass spectrometry.

8. Use of a kit for the *in vitro* diagnosis of arthrosis in an isolated biological sample from a subject, wherein the kit comprises specific antibodies against the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11, or specific oligonucleotides capable of hybridising to the nucleotide sequence that encodes the peptides with the sequences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

9. Use of the kit according to claim 8 wherein further comprises at least one specific antibody against at least one of the peptides with the sequences SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof, or at least one specific oligonucleotide capable of hybridising to the nucleotide sequence that encodes at least one of the peptides with the sequences SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, or combinations thereof.

10. Use of the kit according to any of the claims 8 to 9 wherein the isolated biological sample is a biological fluid, preferably selected from the group consisting of blood, serum, plasma, synovial fluid and urine.

## Patentansprüche

1. Gleichzeitige *In-vitro*-Verwendung des Expressionsniveaus und/oder der Menge der Peptide mit den Sequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11, als Marker für die Diagnose von Arthrose in einer aus einem Probanden isolierten biologischen Probe.

2. *In-vitro*-Verwendung nach Anspruch 1, ferner umfassend das Expressionsniveau und/oder die Menge von mindestens einem der Peptide, ausgewählt aus der Liste bestehend aus: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO:41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79 oder Kombinationen davon.

3. *In-vitro*-Verwendung nach einem der Ansprüche 1 bis 2, wobei die isolierte biologische Probe eine biologische Flüssigkeit ist, vorzugsweise ausgewählt aus der Gruppe bestehend aus Blut Serum, Plasma, Gelenkflüssigkeit und Urin.

4. *In-vitro*-Verfahren zur Diagnose von Arthrose in einer isolierten biologischen Probe eines Probanden, das umfasst:
(a) gleichzeitiges Nachweisen, in der isolierten biologischen Probe des Probanden, des Expressionsniveaus und/oder der Menge der Peptide mit den Sequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11, und
(b) Vergleichen des Expressionsniveaus und/oder der Menge, die in Schritt (a) erhalten werden, mit einer Referenzmenge aus einer isolierten biologischen Probe, die von einem gesunden Kontrollprobanden erhalten wird,
wobei ein signifikant höheres Expressionsniveau und/oder eine signifikant höhere Menge der Peptide in der isolierten biologischen Probe aus dem Probanden von Schritt (a) in Bezug auf das Expressionsniveau und/oder die Menge der Peptide in der isolierten biologischen Probe aus dem gesunden Kontrollprobanden ein Hinweis auf Arthrose ist.

5. Verfahren nach Anspruch 4, ferner umfassend das Bestimmen, in Schritt (a), des Expressionsniveaus und/oder der Menge von mindestens einem der Peptide, ausgewählt aus der Liste bestehend aus: SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79 oder Kombinationen davon,
wobei ein signifikant höheres Expressionsniveau und/oder eine signifikant höhere Menge von mindestens einem der Peptide, ausgewählt aus der Liste bestehend aus: SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77, SEQ ID NO: 78 oder einer beliebigen Kombination davon und/oder ein signifikant niedrigeres Expressionsniveau und/oder eine signifikant niedrigere Menge von mindestens einem der Peptide, ausgewählt aus der Liste bestehend aus: SEQ ID NO: 12, SEQ ID NO: 34, SEQ ID NO: 50, SEQ ID NO: 60, SEQ ID NO: 62, SEQ ID NO: 64, SEQ ID NO: 74 oder beliebigen Kombinationen davon, in der isolierten biologischen Probe aus dem Probanden von Schritt (a) in Bezug auf das Expressionsniveau und/oder die Menge der Peptide in der isolierten biologischen Probe aus dem gesunden Kontrollprobanden ein Hinweis auf Arthrose ist.

6. *In*-*vitro*-Verfahren nach einem der Ansprüche 4 bis 5, wobei die isolierte biologische Probe eine biologische Flüssigkeit ist, vorzugsweise ausgewählt aus der Gruppe bestehend aus Blut Serum, Plasma, Gelenkflüssigkeit und Urin.

7. *In*-*vitro*-Verfahren nach einem der Ansprüche 4 bis 6, wobei der Nachweis der Expressionsniveaus und/oder der Menge der Peptide mittels Massenspektrometrie, ELISA, Array, Microarray, Durchflusszytometrie, Massenspektrometrie, Gaschromatographie-Massenspektrometrie oder Flüssigchromatographie-Massenspektrometrie durchgeführt wird.

8. Verwendung eines Kits für die *In*-*vitro*-Diagnose von Arthrose in einer isolierten biologischen Probe eines Probanden, wobei das Kit spezifische Antikörper gegen die Peptide mit den Sequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11 oder spezifische Oligonukleotide umfasst, die mit der Nukleotidsequenz hybridisieren können, die die Peptide mit den Sequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11 codiert.

9. Verwendung des Kits nach Anspruch 8, ferner umfassend mindestens einen spezifischen Antikörper gegen mindestens eines der Peptide mit den Sequenzen SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79 oder Kombinationen davon oder spezifische Oligonukleotide, die mit der Nukleotidsequenz hybridisieren können, die mindestens eines der Peptide mit den Sequenzen SEQ ID NO: 12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79 oder Kombinationen davon codieren kann.

10. Verwendung des Kits nach einem der Ansprüche 8 bis 9, wobei die isolierte biologische Probe eine biologische Flüssigkeit ist, vorzugsweise ausgewählt aus der Gruppe bestehend aus Blut Serum, Plasma, Gelenkflüssigkeit und Urin.

## Revendications

1. Utilisation simultanée *in vitro* du niveau d'expression et/ou de la quantité des peptides avec les séquences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 et SEQ ID NO: 11, comme marqueur pour le diagnostic de l'arthrose dans un échantillon biologique isolé d'un sujet.

2. Utilisation *in vitro* selon la revendication 1, comprenant en outre le niveau d'expression et/ou la quantité d'au moins l'un des peptides choisis dans la liste constituée de : SEQ ID NO: 12 ; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, ou des combinaisons de celles-ci.

3. Utilisation *in vitro* selon l'une quelconque des revendications 1 à 2, dans laquelle l'échantillon biologique isolé est un fluide biologique, de préférence choisi dans le groupe constitué de sang, sérum, plasma, liquide synovial et urine.

4. Procédé *in vitro* pour le diagnostic de l'arthrose dans un échantillon isolé d'un sujet, qui comprend :
(a) la détection simultanée, dans l'échantillon biologique isolé du sujet, du niveau d'expression et/ou de la quantité de peptides avec les séquences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 et SEQ ID NO: 11, et
(b) la comparaison du niveau d'expression et/ou de la quantité obtenue à l'étape (a) avec une quantité de référence d'un échantillon biologique isolé obtenu d'un sujet témoin sain,
dans lequel un niveau d'expression et/ou une quantité significativement plus élevée des peptides dans l'échantillon biologique isolé du sujet de l'étape (a) par rapport au niveau d'expression et/ou à la quantité des peptides dans l'échantillon biologique isolé du sujet témoin sain, est révélateur d'une arthrose.

5. Procédé selon la revendication 4, comprenant en outre la détermination, à l'étape (a), du niveau d'expression et/ou de la quantité d'au moins l'un des peptides choisis dans la liste constituée de : SEQ ID NO: 12 ; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, ou des combinaisons de celles-ci,
dans lequel un niveau d'expression et/ou une quantité significativement plus élevée d'au moins l'un des peptides choisis dans la liste constituée de : SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 36, SEQ ID NO: 38, SEQ ID NO: 40, SEQ ID NO: 41, SEQ ID NO: 42, SEQ ID NO: 43, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 65, SEQ ID NO: 67, SEQ ID NO: 68, SEQ ID NO: 70, SEQ ID NO: 75, SEQ ID NO: 76, SEQ ID NO: 77, SEQ ID NO: 78, ou toute combinaison de celles-ci, et/ou un niveau d'expression et/ou une quantité significativement plus faible d'au moins l'un des peptides choisis dans la liste constituée de : SEQ ID NO: 12, SEQ ID NO: 34, SEQ ID NO: 50, SEQ ID NO: 60, SEQ ID NO: 62, SEQ ID NO: 64, SEQ ID NO: 74, ou toute combinaison de celles-ci, dans l'échantillon biologique isolé du sujet de l'étape (a) par rapport au niveau d'expression et/ou à la quantité des peptides dans l'échantillon biologique isolé du sujet témoin sain, est révélateur d'une arthrose.

6. Procédé *in vitro* selon l'une quelconque des revendications 4 à 5, dans laquelle l'échantillon biologique isolé est un fluide biologique, de préférence choisi dans le groupe constitué de sang, sérum, plasma, liquide synovial et urine.

7. Procédé *in vitro* selon l'une quelconque des revendications 4 à 6, dans lequel la détection des niveaux d'expression et/ou de la quantité des peptides est effectuée au moyen de spectrométrie de masse, ELISA, réseau, microréseau, cytométrie en flux, spectrométrie de masse, chromatographie en phase gazeuse-spectrométrie de masse ou chromatographie en phase liquide-spectrométrie de masse.

8. Utilisation d'un kit pour le diagnostic *in vitro* de l'arthrose sur un échantillon biologique isolé d'un sujet, dans laquelle le kit comprend des anticorps spécifiques contre les peptides avec les séquences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 et SEQ ID NO: 11, ou des oligonucléotides spécifiques capables de s'hybrider à la séquence nucléotidique qui code les peptides avec les séquences SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 et SEQ ID NO: 11.

9. Utilisation du kit selon la revendication 8 dans laquelle il comprend en outre au moins un anticorps spécifique contre au moins l'un des peptides avec les séquences SEQ ID NO: 12 ; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, ou des combinaisons de celles-ci, ou au moins un oligonucléotide spécifique capable de s'hybrider à la séquence nucléotidique qui code au moins l'un des peptides avec les séquences SEQ ID NO: 12 ; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 24, SEQ ID NO: 28, SEQ ID NO: 32, SEQ ID NO: 33, SEQ ID NO: 34, SEQ ID NO: 36, SEQ ID NO: 38, SEQ ID NO: 40, SEQ ID NO: 41, SEQ ID NO: 42, SEQ ID NO: 43, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 60, SEQ ID NO: 62, SEQ ID NO: 64, SEQ ID NO: 65, SEQ ID NO: 67, SEQ ID NO: 68, SEQ ID NO: 70, SEQ ID NO: 74, SEQ ID NO: 75, SEQ ID NO: 76, SEQ ID NO: 77, SEQ ID NO: 78, SEQ ID NO: 22, SEQ ID NO: 23, SEQ ID NO: 26, SEQ ID NO: 30, SEQ ID NO: 31, SEQ ID NO: 35, SEQ ID NO: 39, SEQ ID NO: 45, SEQ ID NO: 46, SEQ ID NO: 47, SEQ ID NO: 48, SEQ ID NO: 58, SEQ ID NO: 59, SEQ ID NO: 61, SEQ ID NO: 63, SEQ ID NO: 66, SEQ ID NO: 69, SEQ ID NO: 71, SEQ ID NO: 72, SEQ ID NO: 73, SEQ ID NO: 25, SEQ ID NO: 27, SEQ ID NO: 29, SEQ ID NO: 37, SEQ ID NO: 49, SEQ ID NO: 79, ou des combinaisons de celles-ci.

10. Utilisation du kit selon l'une quelconque des revendications 8 à 9 dans laquelle l'échantillon biologique isolé est un fluide biologique, de préférence choisi dans le groupe constitué de sang, sérum, plasma, liquide synovial et urine.


### SEQUENCE LISTING

<110> Fundación Profesor Novoa Santos
<120> METHOD FOR DIAGNOSING ARTHROSIS
<130> PCT2010.2
<150> P201431445
   <151> 2014-10-01
<160> 79
<170> BiSSAP 1.2
<210> 1
   <211> 11
   <212> PRT
   <213> Homo sapiens
<400> 1
   Gln Leu Thr Pro Tyr Ala Gln Arg Met Glu Arg
   1               5                   10
<210> 2
   <211> 12
   <212> PRT
   <213> Homo sapiens
<400> 2
   Gln Lys Trp Glu Ala Glu Pro Val Tyr Val Gln Arg
   1               5                   10
<210> 3
   <211> 14
   <212> PRT
   <213> Homo sapiens
<400> 3
   Glu Leu Gln Gln Arg Leu Glu Pro Tyr Ala Asp Gln Leu Arg
   1               5                   10
<210> 4
   <211> 16
   <212> PRT
   <213> Homo sapiens
<400> 4
   Leu His Glu Leu Gln Glu Lys Leu Ser Pro Leu Gly Glu Glu Met Arg
   1               5                   10                  15
<210> 5
   <211> 18
   <212> PRT
   <213> Homo sapiens
<400> 5
   Gln Lys Leu His Glu Leu Gln Glu Lys Leu Ser Pro Leu Gly Glu Glu
   1               5                   10                  15
   Met Arg
<210> 6
   <211> 18
   <212> PRT
   <213> Homo sapiens
<400> 6
   Leu His Glu Leu Gln Glu Lys Leu Ser Pro Leu Gly Glu Glu Met Arg
   1               5                   10                  15
   Asp Arg
<210> 7
   <211> 19
   <212> PRT
   <213> Homo sapiens
<400> 7
   Asp Ser Phe His Leu Asp Glu Gln Phe Thr Val Pro Val Glu Met Met
   1               5                   10                  15
   Gln Ala Arg
<210> 8
   <211> 20
   <212> PRT
   <213> Homo sapiens
<400> 8
   Ala Arg Ala His Val Asp Ala Leu Arg Thr His Leu Ala Pro Tyr Ser
   1               5                   10                  15
   Asp Glu Leu Arg
               20
<210> 9
   <211> 20
   <212> PRT
   <213> Homo sapiens
<400> 9
   Leu His Glu Leu Gln Glu Lys Leu Ser Pro Leu Gly Glu Glu Met Arg
   1               5                   10                  15
   Asp Arg Ala Arg
               20
<210> 10
   <211> 20
   <212> PRT
   <213> Homo sapiens
<400> 10
   Gln Lys Leu His Glu Leu Gln Glu Lys Leu Ser Pro Leu Gly Glu Glu
   1               5                   10                  15
   Met Arg Asp Arg
   20
<210> 11
   <211> 22
   <212> PRT
   <213> Homo sapiens
<400> 11
   Gln Lys Leu His Glu Leu Gln Glu Lys Leu Ser Pro Leu Gly Glu Glu
   1 5 10 15
   Met Arg Asp Arg Ala Arg
   20
<210> 12
   <211> 7
   <212> PRT
   <213> Homo sapiens
<400> 12
   Val Leu Asn Gln Glu Leu Arg
   1 5
<210> 13
   <211> 7
   <212> PRT
   <213> Homo sapiens
<400> 13
   Tyr Leu Thr Trp Ala Ser Arg
   1 5
<210> 14
   <211> 8
   <212> PRT
   <213> Homo sapiens
<400> 14
   Gly Val Thr Phe Leu Leu Arg Arg
   1 5
<210> 15
   <211> 9
   <212> PRT
   <213> Homo sapiens
<400> 15
   Val Gly Tyr Val Ser Gly Trp Gly Arg
   1 5
<210> 16
   <211> 9
   <212> PRT
   <213> Homo sapiens
<400> 16
   Ala Phe Lys Ala Trp Ala Val Ala Arg
   1 5
<210> 17
   <211> 9
   <212> PRT
   <213> Homo sapiens
<400> 17
   Leu Gln Thr Asp Leu Ser Glu Phe Arg
   1 5
<210> 18
   <211> 10
   <212> PRT
   <213> Homo sapiens
<400> 18
   Gly Phe Ser Pro Lys Asp Val Leu Val Arg
   1 5 10
<210> 19
   <211> 11
   <212> PRT
   <213> Homo sapiens
<400> 19
   Asp Ala Val Ser Ala Ala Phe Lys Gly Leu Lys
   1 5 10
<210> 20
   <211> 10
   <212> PRT
   <213> Homo sapiens
<400> 20
   Trp Leu Gln Gly Ser Gln Glu Leu Pro Arg
   1 5 10
<210> 21
   <211> 10
   <212> PRT
   <213> Homo sapiens
<400> 21
   Leu Glu Thr Pro Asp Phe Gln Leu Phe Lys
   1 5 10
<210> 22
   <211> 10
   <212> PRT
   <213> Homo sapiens
<400> 22
   Leu Gly Met Phe Asn Ile Gln His Cys Lys
   1 5 10
<210> 23
   <211> 11
   <212> PRT
   <213> Homo sapiens
<400> 23
   Leu Ile Cys Gln Ala Thr Gly Phe Ser Pro Arg
   1 5 10
<210> 24
   <211> 11
   <212> PRT
   <213> Homo sapiens
<400> 24
   Leu Arg Cys Leu Ala Pro Leu Glu Gly Ala Arg
   1 5 10
<210> 25
   <211> 10
   <212> PRT
   <213> Homo sapiens
<400> 25
   Leu Gly Met Phe Asn Ile Gln His Cys Lys
   1 5 10
<210> 26
   <211> 9
   <212> PRT
   <213> Homo sapiens
<400> 26
   Trp Gln Glu Glu Met Glu Leu Tyr Arg
   1 5
<210> 27
   <211> 11
   <212> PRT
   <213> Homo sapiens
<400> 27
   Val Gln Pro Tyr Leu Asp Asp Phe Gln Lys Lys
   1 5 10
<210> 28
   <211> 11
   <212> PRT
   <213> Homo sapiens
<400> 28
   Arg Leu Gly Met Phe Asn Ile Gln His Cys Lys
   1 5 10
<210> 29
   <211> 14
   <212> PRT
   <213> Homo sapiens
<400> 29
   Val Val Ala Gly Val Ala Asn Ala Leu Ala His Lys Tyr His
   1 5 10
<210> 30
   <211> 15
   <212> PRT
   <213> Homo sapiens
<400> 30
   Asp Ser Gly Glu Gly Asp Phe Leu Ala Glu Gly Gly Gly Val Arg
   1 5 10 15
<210> 31
   <211> 13
   <212> PRT
   <213> Homo sapiens
<400> 31
   Val Glu Pro Leu Arg Ala Glu Leu Gln Glu Gly Ala Arg
   1 5 10
<210> 32
   <211> 12
   <212> PRT
   <213> Homo sapiens
<400> 32
   Trp Leu Gln Gly Ser Gln Glu Leu Pro Arg Glu Lys
   1 5 10
<210> 33
   <211> 14
   <212> PRT
   <213> Homo sapiens
<400> 33
   Val Cys Pro Phe Ala Gly Ile Leu Glu Asn Gly Ala Val Arg
   1 5 10
<210> 34
   <211> 16
   <212> PRT
   <213> Homo sapiens
<400> 34
   Ala Asp Ser Gly Glu Gly Asp Phe Leu Ala Glu Gly Gly Gly Val Arg
   1 5 10 15
<210> 35
   <211> 12
   <212> PRT
   <213> Homo sapiens
<400> 35
   Ala Leu Tyr Leu Gln Tyr Thr Asp Glu Thr Phe Arg
   1 5 10
<210> 36
   <211> 15
   <212> PRT
   <213> Homo sapiens
<400> 36
   Leu Ala Ala Arg Leu Glu Ala Leu Lys Glu Asn Gly Gly Ala Arg
   1 5 10 15
<210> 37
   <211> 15
   <212> PRT
   <213> Homo sapiens
<400> 37
   Leu Ala Ala Arg Leu Glu Ala Leu Lys Glu Asn Gly Gly Ala Arg
   1 5 10 15
<210> 38
   <211> 13
   <212> PRT
   <213> Homo sapiens
<400> 38
   Thr His Leu Ala Pro Tyr Ser Asp Glu Leu Arg Gln Arg
   1 5 10
<210> 39
   <211> 14
   <212> PRT
   <213> Homo sapiens
<400> 39
   Leu Leu Asp Asn Trp Asp Ser Val Thr Ser Thr Phe Ser Lys
   1               5                   10
<210> 40
   <211> 14
   <212> PRT
   <213> Homo sapiens
<400> 40
   Ile Thr Pro Asn Leu Ala Glu Phe Ala Phe Ser Leu Tyr Arg
   1               5                   10
<210> 41
   <211> 14
   <212> PRT
   <213> Homo sapiens
<400> 41
   Ala Gly Asp Phe Leu Glu Ala Asn Tyr Met Asn Leu Gln Arg
           1               5                   10
<210> 42
   <211> 16
   <212> PRT
   <213> Homo sapiens
<400> 42
   Val Leu Gly Ala Phe Ser Asp Gly Leu Ala His Leu Asp Asn Leu Lys
   1               5                   10                  15
<210> 43
   <211> 15
   <212> PRT
   <213> Homo sapiens
<400> 43
   Gln Lys Val Glu Pro Leu Arg Ala Glu Leu Gln Glu Gly Ala Arg
   1               5                   10                  15
<210> 44
   <211> 14
   <212> PRT
   <213> Homo sapiens
<400> 44
   Tyr Val Met Leu Pro Val Ala Asp Gln Asp Gln Cys Ile Arg
   1 5 10
<210> 45
   <211> 15
   <212> PRT
   <213> Homo sapiens
<400> 45
   Leu Ser Gln Arg Phe Pro Lys Ala Glu Phe Ala Glu Val Ser Lys
   1 5 10 15
<210> 46
   <211> 15
   <212> PRT
   <213> Homo sapiens
<400> 46
   Tyr Ala Ala Ser Ser Tyr Leu Ser Leu Thr Pro Glu Gln Trp Lys
   1 5 10 15
<210> 47
   <211> 15
   <212> PRT
   <213> Homo sapiens
<400> 47
   Tyr Val Gly Gly Gln Glu His Phe Ala His Leu Leu Ile Leu Arg
   1 5 10 15
<210> 48
   <211> 16
   <212> PRT
   <213> Homo sapiens
<400> 48
   Ser Leu Ala Glu Leu Gly Gly His Leu Asp Gln Gln Val Glu Glu Phe
   1 5 10 15
<210> 49
   <211> 17
   <212> PRT
   <213> Homo sapiens
<400> 49
   Gly Leu Pro Gly Pro Pro Asp Val Pro Asp His Ala Ala Tyr His Pro
   1 5 10 15
   Phe
<210> 50
   <211> 15
   <212> PRT
   <213> Homo sapiens
<400> 50
   Ser Tyr Phe Glu Lys Ser Lys Glu Gln Leu Thr Pro Leu Ile Lys
   1 5 10 15
<210> 51
   <211> 17
   <212> PRT
   <213> Homo sapiens
<400> 51
   Asp Ser Gly Arg Asp Tyr Val Ser Gln Phe Glu Gly Ser Ala Leu Gly
   1 5 10 15
   Lys
<210> 52
   <211> 17
   <212> PRT
   <213> Homo sapiens
<400> 52
   Ile Leu Gly Gly His Leu Asp Ala Lys Gly Ser Phe Pro Trp Gln Ala
   1 5 10 15
   Lys
<210> 53
   <211> 16
   <212> PRT
   <213> Homo sapiens
<400> 53
   Val Met Pro Ile Cys Leu Pro Ser Lys Asp Tyr Ala Glu Val Gly Arg
   1 5 10 15
<210> 54
   <211> 19
   <212> PRT
   <213> Homo sapiens
<400> 54
   Glu Val Gln Leu Val Glu Ser Gly Gly Gly Leu Val Gln Pro Gly Gly
   1 5 10 15
   Ser Leu Arg
<210> 55
   <211> 17
   <212> PRT
   <213> Homo sapiens
<400> 55
   Val Tyr Ala Cys Glu Val Thr His Gln Gly Leu Ser Ser Pro Val Thr
   1               5                   10                  15
   Lys
<210> 56
   <211> 17
   <212> PRT
   <213> Homo sapiens
<400> 56
   Val Thr Leu Thr Cys Val Ala Pro Leu Ser Gly Val Asp Phe Gln Leu
   1               5                   10                  15
   Arg
<210> 57
   <211> 18
   <212> PRT
   <213> Homo sapiens
<400> 57
   Glu Ile Val Leu Thr Gln Ser Pro Gly Thr Leu Ser Leu Ser Pro Gly
   1               5                   10                  15
   Glu Arg
<210> 58
   <211> 15
   <212> PRT
   <213> Homo sapiens
<400> 58
   Phe Asn Lys Pro Phe Val Phe Leu Met Ile Glu Gln Asn Thr Lys
   1               5                   10                  15
<210> 59
   <211> 17
   <212> PRT
   <213> Homo sapiens
<400> 59
   Ser Glu Ala Glu Asp Ala Ser Leu Leu Ser Phe Met Gln Gly Tyr Met
   1               5                   10                  15
   Lys
<210> 60
   <211> 17
   <212> PRT
   <213> Homo sapiens
<400> 60
   Ser Glu Ala Glu Asp Ala Ser Leu Leu Ser Phe Met Gln Gly Tyr Met
   1               5                   10                  15
   Lys
<210> 61
   <211> 17
   <212> PRT
   <213> Homo sapiens
<400> 61
   Ser Leu His Thr Leu Phe Gly Asp Lys Leu Cys Thr Val Ala Thr Leu
   1               5                   10                  15
   Arg
<210> 62
   <211> 18
   <212> PRT
   <213> Homo sapiens
<400> 62
   Thr Phe Gly Ser Gly Glu Ala Asp Cys Gly Leu Arg Pro Leu Phe Glu
   1               5                   10                  15
   Lys Lys
<210> 63
   <211> 19
   <212> PRT
   <213> Homo sapiens
<400> 63
   Val Leu Asp Ala Val Arg Gly Ser Pro Ala Ile Asn Val Ala Val His
   1               5                   10                  15
   Val Phe Arg
<210> 64
   <211> 18
   <212> PRT
   <213> Homo sapiens
<400> 64
   Ser Leu Glu Asp Lys Thr Glu Arg Glu Leu Leu Glu Ser Tyr Ile Asp
   1               5                   10                  15
   Gly Arg
<210> 65
   <211> 19
   <212> PRT
   <213> Homo sapiens
<400> 65
   Leu Tyr His Ser Glu Ala Phe Thr Val Asn Phe Gly Asp Thr Glu Glu
   1 5 10 15
   Ala Lys Lys
<210> 66
   <211> 19
   <212> PRT
   <213> Homo sapiens
<400> 66
   Cys Glu Gly Pro Ile Pro Asp Val Thr Phe Glu Leu Leu Arg Glu Gly
   1 5 10 15
   Glu Thr Lys
<210> 67
   <211> 22
   <212> PRT
   <213> Homo sapiens
<400> 67
   Thr Pro Gly Ala Ala Ala Asn Leu Glu Leu Ile Phe Val Gly Pro Gln
   1 5 10 15
   His Ala Gly Asn Tyr Arg
   20
<210> 68
   <211> 20
   <212> PRT
   <213> Homo sapiens
<400> 68
   Gln Ser Asn Asn Lys Tyr Ala Ala Ser Ser Tyr Leu Ser Leu Thr Pro
   1 5 10 15
   Glu Gln Trp Lys
   20
<210> 69
   <211> 21
   <212> PRT
   <213> Homo sapiens
<400> 69
   Ser Glu Ala Glu Asp Ala Ser Leu Leu Ser Phe Met Gln Gly Tyr Met
   1 5 10 15
   Lys His Ala Thr Lys
   20
<210> 70
   <211> 20
   <212> PRT
   <213> Homo sapiens
<400> 70
   Ala His Val Asp Ala Leu Arg Thr His Leu Ala Pro Tyr Ser Asp Glu
   1               5                   10                  15
   Leu Arg Gln Arg
               20
<210> 71
   <211> 20
   <212> PRT
   <213> Homo sapiens
<400> 71
   Leu Thr Pro Tyr Ala Asp Glu Phe Lys Val Lys Ile Asp Gln Thr Val
   1               5                   10                  15
   Glu Glu Leu Arg
               20
<210> 72
   <211> 24
   <212> PRT
   <213> Homo sapiens
<400> 72
   Gly Ala His Gly Pro Arg Pro Asp Thr Val Gly Gln Arg Gly Gly Ser
   1               5                   10                  15
   Arg Pro Ser Pro Gly Pro Ile Arg
               20
<210> 73
   <211> 29
   <212> PRT
   <213> Homo sapiens
<400> 73
   Gly Pro Pro Gly Leu Pro Gly Leu Lys Gly Asp Pro Gly Val Pro Gly
   1               5                   10                  15
   Leu Pro Gly Ala Lys Gly Glu Val Gly Ala Asp Gly Val
               20                  25
<210> 74
   <211> 25
   <212> PRT
   <213> Homo sapiens
<400> 74
   Ala Val Pro Pro Asn Asn Ser Asn Ala Ala Glu Asp Asp Leu Pro Thr
   1               5                   10                  15
   Val Glu Leu Gln Gly Val Val Pro Arg
               20                  25
<210> 75
   <211> 25
   <212> PRT
   <213> Homo sapiens
<400> 75
   Thr Val Arg Thr Pro Gly Ala Ala Ala Asn Leu Glu Leu Ile Phe Val
   1               5                   10                  15
   Gly Pro Gln His Ala Gly Asn Tyr Arg
               20                  25
<210> 76
   <211> 23
   <212> PRT
   <213> Homo sapiens
<400> 76
   Asp Glu Pro Pro Gln Ser Pro Trp Asp Arg Val Lys Asp Leu Ala Thr
   1               5                   10                  15
   Val Tyr Val Asp Val Leu Lys
               20
<210> 77
   <211> 23
   <212> PRT
   <213> Homo sapiens
<400> 77
   Gln Ser Asn Asn Lys Tyr Ala Ala Ser Ser Tyr Leu Ser Leu Thr Pro
   1               5                   10                  15
   Glu Gln Trp Lys Ser His Arg
               20
<210> 78
   <211> 24
   <212> PRT
   <213> Homo sapiens
<400> 78
   Tyr Arg Ser Trp Val Pro His Thr Phe Glu Ser Glu Leu Ser Asp Pro
   1               5                   10                  15
   Val Glu Leu Leu Val Ala Glu Ser
               20
<210> 79
   <211> 28
   <212> PRT
   <213> Homo sapiens
<400> 79
   Ser Ala Ser Leu His Leu Pro Lys Leu Ser Ile Thr Gly Thr Tyr Asp
   1               5                   10                  15
   Leu Lys Ser Val Leu Gly Gln Leu Gly Ile Thr Lys
                   20                  25